# EUROPEAN PATENT SPECIFICATION

(11) **EP 1 948 245 B1**
(45) Date of publication and mention of the grant of the patent: **05.10.2011**
(21) Application number: 06826592.5
(22) Date of filing: 24.10.2006
(51) Int. Cl.: A61K 48/00, A61K 47/36, A61K 9/50, A61K 31/711, A61P 19/10

(54) **COMPOSITIONS AND THEIR USES FOR GENE THERAPY OF BONE CONDITIONS**
ZUSAMMENSETZUNGEN UND IHRE VERWENDUNGEN FÜR DIE GENTHERAPEUTISCHE BEHANDLUNG VON KNOCHENERKRANKUNGEN
COMPOSITIONS ET UTILISATIONS DE CELLES-CI DANS LE CADRE D'UNE THERAPIE GENIQUE POUR TRAITER DES PATHOLOGIES OSSEUSES

(30) Priority: 24.10.2005 US 730123 P
(43) Date of publication of application: 30.07.2008
(73) Proprietor: University of Massachusetts, Boston, MA 02108 (US)
(72) Inventor: GINNS, Edward, I., Shrewsbury, MA 01545-5480 (US); OSTROFF, Gary, R., Worcester, MA 01604 (US)
(74) Representative: Kirkham, Nicholas Andrew
(86) International application number: PCT/US2006/041539
(87) International publication number: WO 2007/050643

(56) References cited:
- WO-A-2006/007372
- WO-A-2006/032039
- WO-A2-2005/018544
- US-A1- 2003 207 827
- US-A1- 2003 216 346
- ERBACHER P ET AL: "CHITOSAN-BASED VECTOR/DNA COMPLEXES FOR GENE DELIVERY: BIOPHYSICAL CHARACTERISTICS AND TRANSFECTION ABILITY" PHARMACEUTICAL RESEARCH, NEW YORK, NY, US, vol. 15, no. 9, 1998, pages 1332-1339, XP002929824 ISSN: 0724-8741
- KIM T H ET AL: "Synergistic effect of poly(ethylenimine) on the transfection efficiency of galactosylated chitosan/DNA complexes" JOURNAL OF CONTROLLED RELEASE, ELSEVIER, AMSTERDAM, NL, vol. 105, no. 3, 20 July 2005 (2005-07-20), pages 354-366, XP004969778 ISSN: 0168-3659
- LOZIER J N ET AL: "EFFICIENT TRANSFECTION OF PRIMARY CELLS IN A CANINE HEMOPHILIA B MODEL USING ADENOVIRUS-POLYLYSINE-DNA COMPLEXES" HUMAN GENE THERAPY, MARY ANN LIEBERT, NEW YORK ,NY, US, vol. 5, no. 3, 1 March 1994 (1994-03-01), pages 313-322, XP000561081 ISSN: 1043-0342
- DATABASE CA [Online] CHEMICAL ABSTRACTS SERVICE, COLUMBUS, OHIO, US; ERBACHER, PATRICK ET AL: "The reduction of the positive charges of polylysine by partial gluconoylation increases the transfection efficiency of polylysine/DNA complexes" XP002116398 retrieved from STN Database accession no. 126:207866 & BIOCHIMICA ET BIOPHYSICA ACTA, BIOMEMBRANES , 1324(1), 27-36 CODEN: BBBMBS; ISSN: 0005-2736, 1997,
- ISHII T ET AL: "Mechanism of cell transfection with plasmid/chitosan complexes" BIOCHIMICA ET BIOPHYSICA ACTA. BIOMEMBRANES, AMSTERDAM, NL, vol. 1514, no. 1, 3 September 2001 (2001-09-03), pages 51-64, XP004300800 ISSN: 0005-2736

### SEQUENCE LISTING

<110> university of Massachusetts
   Ginns, Edward **I.**
   Ostroff, Gary R
<120> COMPOSITIONS AND THEIR USES FOR GENE THERAPY OF BONE CONDITIONS
<130> 20336-00022
<160> 2
<170> Patentin version 3.3
<210> 1
   <211> 1584
   <212> DNA
   <213> Homo sapiens
<400> 1
   aggcaggcga tacttcctgt tgccgggacg ctatatataa cgtgatgagc gcacgggctg     60
   cggagacgca ccggagcgct cgcccagccg ccgcctccaa gcccctgagg tttccgggga    120
   ccacaatgaa caacttgctg tgctgcgcgc tcgtgtttct ggacatctcc attaagtgga    180
   ccacccagga aacgtttcct ccaaagtacc ttcattatga cgaagaaacc tctcatcagc    240
   tgttgtgtga caaatgtcct cctggtacct acctaaaaca acactgtaca gcaaagtgga    300
   agaccgtgtg cgccccttgc cctgaccact actacacaga cagctggcac accagtgacg    360
   agtgtctata ctgcagcccc gtgtgcaagg agctgcagta cgtcaagcag gagtgcaatc    420
   gcacccacaa ccgcgtgtgc gaatgcaagg aagggcgcta ccttgagata gagttctgct    480
   tgaaacatag gagctgccct cctggatttg gagtggtgca agctggaacc ccagagcgaa    540
   atacagtttg caaaagatgt ccagatgggt tcttctcaaa tgagacgtca tctaaagcac    600
   cctgtagaaa acacacaaat tgcagtgtct ttggtctcct gctaactcag aaaggaaatg    660
   caacacacga caacatatgt tccggaaaca gtgaatcaac tcaaaaatgt ggaatagatg    720
   ttaccctgtg tgaggaggca ttcttcaggt ttgctgttcc tacaaagttt acgcctaact    780
   ggcttagtgt cttggtagac aatttgcctg gcaccaaagt aaacgcagag agtgtagaga    840
   ggataaaacg gcaacacagc tcacaagaac agactttcca gctgctgaag ttatggaaac    900
   atcaaaacaa agaccaagat atagtcaaga agatcatcca agatattgac ctctgtgaaa    960
   acagcgtgca gcggcacatt ggacatgcta acctcacctt cgagcagctt cgtagcttga   1020
   tggaaagctt accgggaaag aaagtgggag cagaagacat tgaaaaaaca ataaaggcat   1080
   gcaaacccag tgaccagatc ctgaagctgc tcagtttgtg gcgaataaaa aatggcgacc   1140
   aagacacctt gaagggccta atgcacgcac taaagcactc aaagacgtac cactttccca   1200
   aaactgtcac tcagagtcta aagaagacca tcaggttcct tcacagcttc acaatgtaca   1260
   aattgtatca gaagttattt ttagaaatga taggtaacca ggtccaatca gtaaaaataa   1320
   gctgcttata actggaaatg gccattgagc tgtttcctca caattggcga gatcccatgg   1380
   atgagtaaac tgtttctcag gcacttgagg ctttcagtga tatctttctc attaccagtg   1440
   actaattttg ccacagggta ctaaaagaaa ctatgatgtg gagaaaggac taacatctcc   1500
   tccaataaac cccaaatggt taatccaact gtcagatctg gatcgttatc tactgactat   1560
   attttccctt attactgctt gcag                                          1584
<210> 2
   <211> 401
   <212> PRT
   <213> Homo sapiens
<220>
   <221> DOMAIN
   <222> (28)..(124)
   <223> Tumor necrosis factor receptor (TNFR) domain
<220>
   <221> DOMAIN
   <222> (124)..(185)
   <223> Tumor necrosis factor receptor (TNFR) domain
<400> 2
   Met Asn Asn Leu Leu Cys Cys Ala Leu Val Phe Leu Asp Ile Ser Ile
   1               5                   10                  15
   Lys Trp Thr Thr Gln Glu Thr Phe Pro Pro Lys Tyr Leu His Tyr Asp
               20                  25                  30
   Glu Glu Thr Ser His Gln Leu Leu Cys Asp Lys Cys Pro Pro Gly Thr
           35                  40                  45
   Tyr Leu Lys Gln His Cys Thr Ala Lys Trp Lys Thr Val Cys Ala Pro
       50                  55                  60
   Cys Pro Asp His Tyr Tyr Thr Asp Ser Trp His Thr Ser Asp Glu Cys
   65                  70                  75                  80
   Leu Tyr Cys Ser Pro Val Cys Lys Glu Leu Gln Tyr Val Lys Gln Glu
                   85                  90                  95
   Cys Asn Arg Thr His Asn Arg Val Cys Glu Cys Lys Glu Gly Arg Tyr
               100                 105                 110
   Leu Glu Ile Glu Phe Cys Leu Lys His Arg Ser Cys Pro Pro Gly Phe
           115                 120                 125
   Gly Val Val Gln Ala Gly Thr Pro Glu Arg Asn Thr Val Cys Lys Arg
       130                 135                 140
   Cys Pro Asp Gly Phe Phe Ser Asn Glu Thr Ser Ser Lys Ala Pro Cys
   145                 150                 155                 160
   Arg Lys His Thr Asn Cys Ser Val Phe Gly Leu Leu Leu Thr Gln Lys
                   165                 170                 175
   Gly Asn Ala Thr His Asp Asn Ile Cys Ser Gly Asn Ser Glu Ser Thr
                   180                     185                     190
   Gln Lys Cys Gly Ile Asp Val Thr Leu Cys Glu Glu Ala Phe Phe Arg
           195                 200                 205
   Phe Ala Val Pro Thr Lys Phe Thr Pro Asn Trp Leu Ser Val Leu Val
       210                 215                 220
   Asp Asn Leu Pro Gly Thr Lys Val Asn Ala Glu Ser Val Glu Arg Ile
   225                 230                 235                     240
   Lys Arg Gln His Ser Ser Gln Glu Gln Thr Phe Gln Leu Leu Lys Leu
                   245                 250                 255
   Trp Lys His Gln Asn Lys Asp Gln Asp Ile Val Lys Lys Ile Ile Gln
               260                 265                 270
   Asp Ile Asp Leu Cys Glu Asn Ser Val Gln Arg His Ile Gly His Ala
           275                 280                 285
   Asn Leu Thr Phe Glu Gln Leu Arg Ser Leu Met Glu Ser Leu Pro Gly
       290                 295                 300
   Lys Lys Val Gly Ala Glu Asp Ile Glu Lys Thr Ile Lys Ala Cys Lys
   305                 310                 315                     320
   Pro Ser Asp Gln Ile Leu Lys Leu Leu Ser Leu Trp Arg Ile Lys Asn
                   325                 330                 335
   Gly Asp Gln Asp Thr Leu Lys Gly Leu Met His Ala Leu Lys His Ser
               340                 345                 350
   Lys Thr Tyr His Phe Pro Lys Thr Val Thr Gln Ser Leu Lys Lys Thr
           355                 360                 365
   Ile Arg Phe Leu His Ser Phe Thr Met Tyr Lys Leu Tyr Gln Lys Leu
       370                 375                 380
   Phe Leu Glu Met Ile Gly Asn Gln Val Gln Ser Val Lys Ile Ser Cys
   385                 390                 395                     400
   Leu

## Description

### FIELD OF THE INVENTION

The present invention relates to compositions for the treatment of low bone density. The present invention also relates to compositions for the treatment of ostboprotegeon-dependent conditions.

### BACKGROUND OF THE INVENTION

The Surgeon General's Report on Bone Health and Osteoporosis estimates that jn 2020 approximately half of Americans over age 50 will have or will be at risk for developing osteoporosis. The National Osteoporosis Foundation has estimated that there are about 10 million active cases of osteoporosis in the United States, 8 million women and 2 million men, with an additional 34 million Americans at serious risk of osteoporosis due to low bone mass. Osteoporosis is responsible for more than 1.5 million fractures annually, including over 300,000 hip fractures; approximately 700,000 vertebral fractures; 250,000 wrist fractures; and 300,000 fractures at other sites. The estimated national direct expenditures (hospitals and nursing homes) for osteoporotic hip fractures were $18 billion dollars in 2002. Patients with hip fractures are much more likely to experience additional fractures in the future. The loss of quality of life underlying these statistics is difficult to overstate. In addition, acute and site-specific low bone density conditions include inflammation mediated osteolysis, tumor-induced osteolysis, prosthetic implant loosening, periodonitis or osteoarthritis.

Cell Biology of Bone Homeostasis. Bone is a dynamic tissue that undergoes constant remodeling (resorption and replacement) in the skeleton. The principal cell types responsible for skeletal maintenance are the resorptive osteoclasts and bone-synthesizing osteoblasts, both of which are influenced by hormones, growth factors and inflammatory mediators. Bone formation by mesenchymal stem cell derived osteoblasts, and its modeling and remodeling by osteoclasts arising from hematopoietic precursors of the monocyte/macrophage lineage, is a tightly regulate system (Huang, W., et,al., A rapid multiparameter approach to study factors that regulate osteoclastogenesis: Demonstration of the combinatorial dominant effects of TNF-a and TGF-ß in RANKL-mediated osteoclastogenesis. Calcif. Tissue Int. 73:584-593 (2003). Maintenance of normal bone mass is dependent on the homeostatic complex balance between formation and resorption, involving both local and systemic factors and signals. When there is an imbalance between these two processes, either increased (osteopetrosis) or decreased (osteoporosis) bone density occurs. Chronic low bone density is seen in postmenopausal, age-related and inflammatory diseases, while acute low bone density is observed in prosthesis loosening or tumor-induced osteolysis.

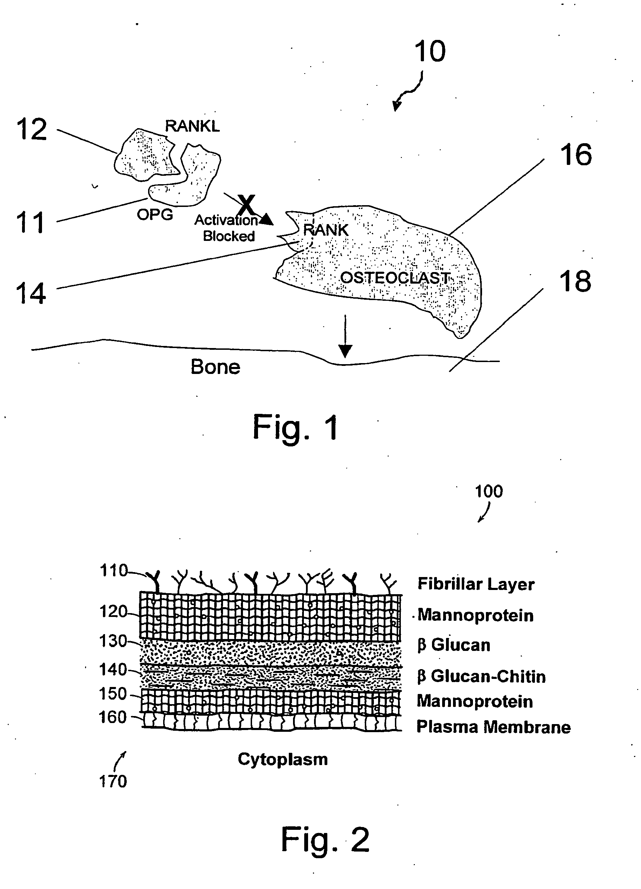

Osteoclasts. Osteoclasts arise from hematopoietic precursors of the monocyte/macrophage lineage F4-80 positive cells in response to specific signals (Boyle, W. J., et al., Osteoclast differentiation and activation. Nature 423, 337-342 (2003). Two growth factors are required for this to occur, colony-stimulating factor-1 (CSF-1; M-CSF) and the TNF superfamily member RANKL (receptor activator of NF-κB ligand; also called TRANCE, OPGL, and ODF. Fig. 1 is a schematic diagram of the signaling mechanisms involved in osteoclast differentiation, where RANKL activates osteoclast differentiation by activating its receptor RANK, while osteoprotegerin (OPG, also known as osteoclastogenesis inhibitory factor) sequesters RANKL blocking its binding to the cell surface.

In the skeleton, both CSF-1 and RANKL are supplied by osteoblasts, although there are additional cellular sources in other tissues. CSF-1 binds to pre-osteoclasts via its receptor, the proto-oncogene c-Fms, and stimulates the expression of the RANKL receptor, RANK, rendering those cells responsive to RANKL. Activation of RANK stimulates expression of NF-κB-dependent genes via the RANK-associated factor TRAF6 and also activates the Jun kinase and phosphoinositol pathways. Together, these pathways inhibit apoptosis and initiate a host of other cellular responses that prepare the osteoclast to resorb bone. These include chemokine-induced chemotaxis to sites of resorption, cell fusion to produce multi-nucleated cells, formation of a specialized actin ring that promotes attachment to bone via αvβ3 integrins, expression of proteases and proton pumps to dissolve bone matrix, and development of extremely active vesicular transport to secrete degradative molecules and to ingest and transport the dissolved bone matrix. RANKL is an attractive potential target for regulating osteoclast activity, acting upstream of these multiple differentiation steps to inhibit their differentiation in vivo.

The RANKL/RANK/OPG pathway signaling may also be important in vascular physiology and pathology with regard to endothelial cell survival, angiogenesis, monocyte or endothelial cell recruitment, and smooth muscle cell osteogenesis and calcification. The results of studies suggest that RANKL could promote while OPG could protect against vascular calcification coincident with decreases in bone mineralization with aging, osteoporosis or disease (Collin-Osdoby, P., Regulation of vascular calcification by osteoclast regulatory factors RANKL and osteoprotegerin. Circulation Res. 2004 95(11):1046-1057).

Osteoprotegerin. OPG is a member of the tumor necrosis factor receptor (TNFR) superfamily, and is a secreted basic 401 amino acid glycoprotein that exists in a monomeric form of about 60-kD and a disulfide-linked homodimeric form of about 120 kD. OPG is produced by osteoblasts and marrow stromal cells. OPG blocks osteoclastogenesis in a dose dependent manner by functioning as a soluble "decoy" receptor that prevents RANKL from binding to RANK (Fig. 1). See Schoppet, M., et al., RANK ligand and osteoprotegerin: paracrine regulatory of bone metabolism and vascular function, Arterioscler Thromb Vasc Biol. 2002 Apr 1;22 (4):549-53. Osteoprotegerin was reported in 1997 by Simonet et al. who identified and characterized it as a secreted member of the tumor necrosis factor receptor (TNFR) superfamily that had protective bone effects *in vitro* and *in vivo* (Simonet, W. S., et al. Osteoprotegerin: A Novel Secreted Protein Involved in the Regulation of Bone Density. Cell 1997 89, 309-319). Both intravenous injection of recombinant OPG protein and transgenic overexpression of OPG in OPG(-/-) mice effectively rescue the osteoporotic bone phenotype observed in OPG-deficient mice. See Min, H., et al., Osteoprotegerin reverses osteoporosis by inhibiting endosteal osteoclasts and prevents vascular calcification by blocking a process resembling osteoclastogenesis. J Exp Med 2000 192, 463-474.

Over-expression of OPG in transgenic mice has been demonstrated to result in increased skeletal mass and reduced osteoclast number and activity, presumably by blocking RANKL/RANK interaction (Simonet, W. S., *et al.* (1997), while the deficiency of OPG results in osteoporosis. See Bucay, N., et al., Osteoprotegerin-deficient mice develop early onset osteoporosis and arterial calcification. Genes Dev 1998 12, 1260-1268, and Mizuno, A., et al,. Severe osteoporosis in mice lacking osteoclastogenesis inhibitory factor/osteoprotegerin. Biochem Biophys Res Commun 1998 247, 610-615.

In addition to osteoporosis, several other bones conditions are associated with lost of bone mass, including periprosthetic osteolysis (Yang, S.Y., et al., Adeno-associated virus-mediated osteoprotegerin gene transfer protects against particulate polyethylene-induced osteolysis in a murine model, Arthritis Rheum. 2002 Sep; 46(9):2514-23), osteolysis associated with tumor metastasis, juvenile Paget's disease, Gaucher disease, antiviral treatment of HIV, disuse osteopenia, thalasemia and inflammatory bowel disease.

Bone cancer pain most commonly occurs when tumors originating in breast, prostate, or lung metastasize to long bones, spinal vertebrae, and/or pelvis. Primary and metastatic cancers involving bone account for approximately 400,000 new cancer cases per year in the United States alone, and >70% of patients with advanced breast or prostate cancer have skeletal metastases. Reported results of studies in animal models of bone pain have indicated that osteoprotegerin treatment halted further bone destruction, reduced ongoing and movement-evoked pain, and reversed several aspects of the neurochemical reorganization of the spinal cord. See Luger, N.M., et al., Osteoprotegerin diminishes advanced bone cancer pain, Cancer Res. 2001 May 15; 61 (10):4038-47.

Therapeutic approaches to correct low bone density are directed at either inhibiting bone resorption or stimulating bone formation. While the majority of currently approved drugs for treatment and prevention of low bone density act to increase bone mass by inhibiting osteoclastic bone resorption, bisphosphonates, estrogens, salmon calcitonin and the selective estrogen receptor modulator raloxifene, discussed below, there has recently been a rapid growth of interest in exploring anabolic drugs, including bone morphogenetic proteins and statins. Hormone therapy may also be used, including estrogen or parathyroid hormone. Although these therapies have been in clinical usage, for decades in the case of bisphosphonates and estrogen, their limited efficacy is evident when one considers the persistence of widespread osteoporosis in the aging population.

Bisphosphonates such as aledronate, risedronate, ibandronic acid and others are incorporated into the bone hydroxyapatite mineral and act to inhibit bone resorption by multiple mechanisms, including: a) interfering with osteoclast bone attachment, b) inhibiting differentiation of osteoclast precursors, and c) inhibiting osteoclast function following their selectively uptake by osteoclasts. Estrogen has been widely used for treatment of osteoporosis in postmenopausal women for many years. Although the mechanism of estrogen action still needs further investigation, studies have demonstrated that estrogen replacement in postmenopausal women reduces skeletal remodeling, and attenuates the loss and can even increase both trabecular and cortical bone mass. Despite the beneficial effects of estrogen therapy on bone density in postmenopausal women, its use is associated with an increased risk of breast and uterine cancer, and causes vaginal bleeding, breast tenderness and bloating. Selective Estrogen Receptor Modulators were developed for treatment of osteoporosis because of the complications and risks of estrogen therapy. Like estrogen, SERMs such as tamoxifen and raloxifene are agonists for estrogen receptors in bone, but are estrogen receptor antagonists in breast tissue.

Several clinical trials have suggested that intranasal salmon calcitonin therapy is effective at preventing the loss of bone mass and at diminishing the rate of vertebral fractures. Salmon calcitonin has been shown to reduce the risk of vertebral fractures by 36% in postmenopausal women with osteoporosis and previous fractures, with a safety profile comparable to placebo over long-term use. Salmon calcitonin is well tolerated, provides some analgesia in the case of fractures, and is a reasonable alternative to hormone therapy.

The discovery of the RANKL/OPG/RANK pathway has opened up new opportunities to develop improved anti-resorptive therapies. As noted above, constitutive overexpression of OPG in transgenic mice led to mild osteopetrosis and OPG -/- mice are severely osteoporotic. Transgenic overexpression of OPG rescues the knockout phenotype (Min, H., et al., 2000). Inhibition of the RANKL pathway, by either direct RANKL inhibition, or by increasing the level of soluble OPG, to reduce osteoclastic bone resorption is a promising paradigm for osteoporosis treatment.

Bone Morphogenetic Proteins. Recently there has been a rapid growth of interest in anabolic approaches, for example, the use bone morphogenetic proteins (BMPs) or of IV infusion of pulsatile doses of parathyroid hormone. These therapeutic strategies have great promise, and initial assessments of BMP's are encouraging. However, these therapeutic approaches are not without potential risks of bone overgrowth, osteophytes, ectopic bone, vascular calcification, or even neoplasms.

Monoclonal Antibodies. In a recent small scale clinical trial, a single injection of a monoclonal human antibody to RANKL was shown to decrease bone turnover markers for up to six months (Bekker, P. J., et al., A single-dose placebo-controlled study of AMG 162, a fully human monoclonal antibody to RANKL, in postmenopausal women. J Bone Miner Res 2004 19, 1059-1066). Adalimumab (Humira®), a human monoclonal anti-tumor necrosis factor (TNF) antibody, effectively reduces the symptoms and signs of rheumatoid arthritis and prevents progression of erosive joint changes seen on radiological examination.

Statins. In a study of a large cohort of mostly male veterans, statin use was associated with a 36 percent reduction in fracture risk compared with no lipid-lowering therapy, and a 32 percent risk reduction when compared with other lipid-lowering therapies. Several biological mechanisms have been proposed to explain an association between statins and bone health, including reduced inflammation and promotion of new bone growth through improvements in small blood vessel function (Scranton, R.E. (2005). Statin use and fracture risk: study of a US veterans population. Arch. Intern. Med. 165: 2007-2012).

Osteoprotegerin Gene Therapy Using Viral Vectors. Osteoprotegerin is a protein which prevents bone resorption by inhibition of osteoclastogenesis, function, and survival, and these activities have made recombinant OPG an attractive drug candidate for the treatment of chronic bone resorptive diseases such as osteoporosis. Gene therapy has the potential to achieve long-term treatment by delivering genes of anti-resorptive proteins. OPG has been delivered by adeno associated virus, and adenovirus, either as DNA encoding only OPG or as a fusion protein with the IgG Fc chain. *In vivo* administration of rAA V-OPG-IRES-EGFP resulted in detectable transduction of myocytes at the injection site and a significant increase in expression of serum OPG levels two days after injection, with decreased fracture remodeling, but with little influence on the structural strength of healing fractures.

Extracted yeast cell wall particles are readily available, biodegradable, substantially spherical particles about 2-4 µm in diameter. Preparation of extracted yeast cell wall particles is known in the art, and is described, for example in U.S. Pat. Nos. 4,992,540; 5,082,936; 5,028,703; 5,032,401; 5,322,841; 5,401,727; 5,504,079; 5,968,811; 6,444,448 B1; 6,476,003 B1; published U.S. applications 2003/0216346 A1, 2004/0014715 A1, and PCT published application WO 02/12348 A2. A form of extracted yeast cell wall particles, referred to as "whole glucan particles," have been suggested as delivery vehicles, but have been limited either to release by simple diffusion of active ingredient from the particle or release of an agent chemically crosslinked to the whole glucan particle by biodegradation of the particle matrix. See U.S. Pat. Nos.5,032,401 and 5,607,677. An improved yeast cell wall drug delivery system is disclosed in U.S. published patent application US2005281781 and published PCT international patent application WO2006007372 A3 overcomes these limitations. "Yeast cell wall particle" (YCWP) encompasses yeast glucan particles (YGP) and yeast glucan-mannan particles (YGMP).

Another important component of the GI immune system is the M or microfold cell. M cells are a specific cell type in the intestinal epithelium over lymphoid follicles that endocytose a variety of protein and peptide antigens. Instead of digesting these proteins, M cells transport them into the underlying tissue, where they are taken up by local dendritic cells and macrophages.

M cells take up molecules and particles from the gut lumen by endocytosis or phagocytosis. This material is then transported through the interior of the cell in vesicles to the basal cell membrane, where it is released into the extracellular space. This process is known as transcytosis. At their basal surface, the cell membrane of M cells is extensively folded around underlying lymphocytes and antigen-presenting cells, which take up the transported material released from the M cells and process it for antigen presentation.

A study has shown that transcytosis of yeast particles (3.4 +/- 0.8 micron in diameter) by M cells of the Peyer's patches takes less than 1 hour (Beier, R., & Gebert, A., Kinetics of particle uptake in the domes of Peyer's patches, Am J Physiol. 1998 Jul; 275(1 Pt 1):G130-7). Without significant phagocytosis by intraepithelial macrophages, the yeast particles migrate down to and across the basal lamina within 2.5-4 hours, where they quickly get phagocytosed and transported out of the Peyer's patch domes. M cells found in human nasopharyngeal lymphoid tissue (tonsils and adenoids) have been shown to be involved in the sampling of viruses that cause respiratory infections. Studies of an *in vitro* M cells model have shown uptake of fluorescently labeled microspheres (Fluospheres, 0.2 µm) and chitosan microparticles (0.2 µm) van der Lubben I.M., et al., Transport of chitosan microparticles for mucosal vaccine delivery in a human intestinal M-cell model, J Drug Target, 2002 Sep; 10(6):449-56. A lectin, *Ulex europaeus* agglutinin I (UEA1, specific for alpha-L-fucose residues) has been used to target either polystyrene microspheres (0.5 µm) or polymerized liposomes to M cells (0.2 µm) (Clark, M.A., et al., Targeting polymerised liposome vaccine carriers to intestinal M cells, Vaccine, 2001 Oct 12;20(1-2):208-17). *In vivo* studies in mice have reported that poly-D,L-lactic acid (PDLLA) microspheres or gelatin microspheres (GM) can be efficiently taken up by macrophages and M cells. (Nakase, H., et al., Biodegradable microspheres targeting mucosal immune-regulating cells: new approach for treatment of inflammatory bowel disease, J Gastroenterol. 2003 Mar; 38 Suppl 15:59-62).

However, it has been reported that uptake of synthetic particulate delivery vehicles including poly (DL-lactide-co-glycolide) microparticles and liposomes is highly variable, and is determined by the physical properties of both particles and M cells. Clark, M.A., et al., Exploiting M cells for drug and vaccine delivery, Adv Drug Deliv Rev. 2001 Aug 23;50(1-2):81-106. The same study reported that delivery may be enhanced by coating the particles or liposomes with reagents including appropriate lectins, microbial adhesins and immunoglobulins which selectively bind to M cell surfaces. See also, Florence, A.T., The oral absorption of micro- and nanoparticulates: neither exceptional nor unusual, Pharm Res. 1997 Mar;14(3):259-66.

Pathogen pattern recognition receptors (PRRs) recognize common structural and molecular motifs present on microbial surfaces and contribute to induction of innate immune responses. Mannose receptors and beta-glucan receptors in part participate in the recognition of fungal pathogens. The mannose receptor (MR), a carbohydrate-binding receptor expressed on subsets of macrophages, is considered one such PRR. Macrophages have receptors for both mannose and mannose-6-phosphate that can bind to and internalize molecules displaying these sugars. The molecules are internalized by endocytosis into a pre-lysosomal endosome. This internalization has been used to enhance entry of oligonucleotides into macrophages using bovine serum albumin modified with mannose-6-phosphate and linked to an oligodeoxynucleotide by a disulfide bridge to a modified 3' end; see Bonfils, E., et al., Nucl. Acids Res. 1992 20, 4621-4629. Macrophages also express beta-glucan receptors, including CR3 (Ross, G.D., et al., Specificity of membrane complement receptor type three (CR3) for β-glucans. Complement Inflamm. 1987 4:61), dectin-1. (Brown, G.D. and S. Gordon. Immune recognition. A new receptor for β-glucans. Nature 2001 413:36.), and lactosylceramide (Zimmerman J.W., et al., A novel carbohydrate-glycosphinglipid interaction between a beta-(1-3)-glucan immunomodulator, PGG-glucan, and lactosylceramide of human leukocytes. J Biol Chem. 1998 273(34):22014-20). The beta-glucan receptor, CR₃ is predominantly expressed on monocytes, neutrophils and NK cells, whereas dectin-1 is predominantly expressed on the surface of cells of the macrophages. Lactosylceramide is found at high levels in M cells. Microglia can also express a beta-glucan receptor (Muller, C.D., et al. Functional beta-glucan receptor expression by a microglial cell line, Res Immunol. 1994 145(4):267-75).

There is evidence for additive effects on phagocytosis of binding to both mannose and beta-glucan receptors. Giaimis et al. reported observations suggesting that phagocytosis of unopsonized heat-killed yeast (*S. cerevisiae*) by murine macrophage-like cell lines as well as murine peritoneal resident macrophages is mediated by both mannose and beta-glucan receptors. To achieve maximal phagocytosis of unopsonized heat-killed yeast, coexpression of both mannose and beta-glucan receptors is required (Giaimis, J., et al., Both mannose and beta-glucan receptors are involved in phagocytosis of unopsonized, heat-killed Saccharomyces cerevisiae by murine macrophages, J Leukoc Biol. 1993 54(6):564-71).

### SUMMARY OF THE INVENTION

In certain preferred embodiments, the present invention provides compositions for the treatment of bone conditions associated with loss of bone. In preferred embodiments, the present invention provides compositions for the treatment of osteoptotegrin-responaive conditions. In preferred embodiments, the treatment is mediated by macrophage-targeted expression of an osteoprotogerin or a functional equivalent thereof by oral administration using the compositions and methods of the present invention. In preferred embodiments, plasmid DNAs expressing an osteoprotegerin or a functional equivalent thereof are incorporated into compositions that include yeast glucan particles (YGP) or yeast glucan-mannan particles (YGMP) in the form of cationic polymer-DNA nanocomplexes. These YGP-DNA and YGMP-DNA microparticles are systemically, mucosally and orally bioavailable through receptor mediated uptake into tissue, mucosal and gut associated lymphatic tissue (GALT) macrophages via carbohydrate receptor binding to the particle surface glucan and mannan polysaccharides. Upon phagocytosis the particles are engulfed into an endosomal compartment where the cationic polymer releases the DNA and swells the endosome releasing the DNA into the cytoplasm. Incorporation of excipients into the YGP-DNA and YGMP-DNA formulations facilitate endosomal DNA release and nuclear uptake.

The invention provides a composition according to any one of claims 1 to 6. In particularly preferred embodiments, the recombinant DNA construct is an expression vector comprising a control element operatively linked to an open reading frame encoding an osteoprotegerin or a functional equivalent thereof. In certain embodiments, the expression vector is pIRBS2DsRED2-hOPG. In other embodiments, the expression vector includes the polynucleotide of SEQ ID NO: 1. In other embodiments, the expression vector encodes a polypeptide selected from the group consisting of the polypeptide of SEQ ID NO: 2, a polypeptide consisting essentially of residues 28 to 124 of SEQ ID NO: 2, a polypeptide consisting essentially of residues 124 to 185 of SEQ ID NO: 2, and a polypeptide consisting essentially of residues 28 to 185 of SEQ ID NO: 2. The carrier is an extracted yeast cell wall defining an internal space and comprising 6 to about 90 weight percent beta-glucan.

The invention provides a method of treating a condition characterized by low bone density in a subject in need of treatment, comprising the step of providing the above composition and a pharmaceutically acceptable-excipient in an oral, buccal, sublingual, pulmonary or transmucosal dosage form. The method includes the step of administering an effective amount of the composition to the subject The condition can be osteoporosis, periprosthetic osteolysis, disuse osteopenia, arterial calcification, or osteolysis associated with tumor metastasis, bone cancer pain, juvenile Paget's disease, Gaucher disease, antiviral treatment of HIV, arthritis, thalasemia or inflammatory bowel disease.

In further embodiments, the invention is useful in a method of increasing osteoprotegarin expression in a cell comprising the steps of providing the composition of the invention and contacting the cell with the composition. Generally, the cell is a macrophage, an osteoclast, an osteoclast precursor, an M cell of a Peyer's patch, a monocyte, a neutrophil, a dendritic cell, a Langerhans cell, a Kupffer cell, an alveolar phagocyte, a peritoneal macrophage, a milk macrophage, a microglial cell, an eosinophil, a granulocytes, a mesengial phagocyte or a synovial A cell. In preferred, embodiments, the method further includes the step of expressing an osteoprotegerin in the cell. In preferred embodiments, the method further includes the step of secreting the osteoprotegerin from the cell. The secreted osteoprotegerin is present in a concentration of at least 2 pmole/l in the extracellular fluid, preferably in the extracellular fluid in contact with the cell.

In other aspects, the composition can be used for the manufacture of a medicament for the treatment of a condition characterized by low bone density. The condition can be osteoporosis, periprosthetic osteolysis, disuse osteopenia, arterial calcification, or osteolysis associated with tumor metastasis, bone cancer pain, juvenile Paget's disease, Gaucher disease, antiviral treatment ofHIV, arthritis, thalasemia or inflammatory bowel disease.

In further embodiments, the invention is useful in a method of increasing osteoprotegerin expression in a cell, including the steps of providing an effective amount of a delivery system comprising the composition of any one of claims 1 to 6. including contacting the cell with the delivery system; and expressing the osteoprotegerin. The step of contacting may be performed *in vitro* or *in vivo*. Preferably, the recombinant DNA construct is an expression vector comprising a control element operatively linked to an open reading frame encoding an osteoprotegerin or a functional equivalent thereof, such as pIRES2DsRBD2-hOPG. In certain embodiments, the expression vector includes the polynucleotide of SEQ ID NO: 1. In preferred embodiments, the expression vector encodes a polypeptide selected from the group consisting of the polypeptide of SEQ ID NO: 2, a polypeptide consisting essentially of residues 28 to 124 of SEQ ID NO: 2, a polypeptide consisting essentially of residues 124 to 185 of SEQ ID NO: 2, and a polypeptide consisting essentially of residues 28 to 185 of SEQ ID NO: 2. Generally, the cell is a macrophage, an osteoclast, an osteoclast precursor, an M cell of a Peyer's patch, a monocyte, a neutrophil, a dendritic cell, a Langerhans cell, a Kupffer cell, an alveolar phagocyte, a peritoneal macrophage, a milk macrophage, a microglial cell, an eosinophil, a granulocytes, a mesengial phagocyte or a synovial A cell.

In further embodiments, the invention is useful in a method of treating of an osteoprotegerin-responsive condition in a subject in need of treatment including the step of providing the composition of the invention and a pharmaceutically acceptable excipient in an oral, buccal, sublingual, pulmonary or transmucosal dosage form. Typically, the method also includes the step of administering an effective amount of the composition to the subject Generally, the condition is osteoporosis, periprosthetic osteolysis, disuse osteopenia, arterial calcification, or osteolysis associated with tumor metastasis, bone cancer pain, juvenile Paget's disease, Gaucher disease, antiviral treatment of HIV, arthritis, thalasemia or inflammatory bowel disease.

In yet further embodiments, the invention provides a method of making an osteoprotegarin delivery system according to claim 17.

In certain preferred embodiments, the protein encoded by the open reading frame is a protein that produces a therapeutic effect in a subject having osteoporosis, periprosthetic osteolysis, disuse osteopenia, arterial calcification, or osteolysis associated with tumor metastasis, bone cancer pain, juvenile Paget's disease, gauche disease, antiviral treatment of HIV, arthritis, thalasemia or inflammatory bowel disease. In particularly preferred embodiments, the protein encoded by the open reading frame is human osteoprotegerin or its functional equivalent.

In other embodiments, the invention provides a pharmaceutical composition comprising an osteoprotegerin or functional equivalent and a pharmaceutically acceptable excipient. In preferred embodiments, the composition is suitable for oral administration. In other preferred embodiments, the composition is formulated for parenteral administration, most preferably for subcutaneous or intramuscular administration. In other preferred embodiments, the composition is formulated for mucosal administration.

The present invention is useful in a method of treating a condition associated with low bone density including the steps of providing an effective amount of a therapeutic delivery system comprising an extracted yeast cell wall comprising beta-glucan, a payload trapping molecule and a payload molecule, wherein the payload molecule is an expression vector comprising a control element operatively linked to an open reading frame encoding a deficient bone protein, such as osteoprotegerin; and contacting a cell having such a bone protein deficiency with the therapeutic delivery system. The step of contacting the cell can be performed *in vitro* or *in vivo.* In preferred embodiments, the therapeutic delivery system is internalized by the cell, typically by phagocytosis.

The cell that can be suitably treated can be a macrophage, an M cell of a Peyer's patch, a monocyte, a neutrophil, a dendritic cell, a Langerhans cell, a Kupffer cell, an alveolar phagocyte, a peritoneal macrophage, a milk macrophage, a microglial cell, an eosinophil, a granulocytes, a mesengial phagocyte or a synovial A cell. In certain preferred embodiments, the cell is an osteoclast or an osteoclast precursor.

The foregoing and other features and advantages of the particulate drug delivery system and methods will be apparent from the following more particular description of preferred embodiments of the system and method as illustrated in the accompanying drawings in which like reference characters refer to the same parts throughout the different views.

### BRIEF DESCRIPTION OF THE DRAWINGS

Fig. 1 is a schematic diagram 10 of the signaling, mechanisms involved in osteoclast 16 differentiation, where RANKL ("Receptor Activator of NF-KappaB Ligand") 12 activates osteoclast differentiation by activating its receptor RANK ("Receptor Activator of NF-KappaB") 14, which is inhibited by OPG 11 sequestering RANKL and blocking its binding to the osteoblast cell surface and subsequent osteoblast action on bone 18.
Fig. 2 is a schematic diagram 100 of a transverse section of a yeast cell wall, showing, from outside to inside, an outer fibrillar layer 110, an outer mannoprotein layer 120, a beta glucan layer 130, a beta glucan layer - chitin layer 140, an inner mannoprotein layer 150, the plasma membrane 160 and the cytoplasm 170.

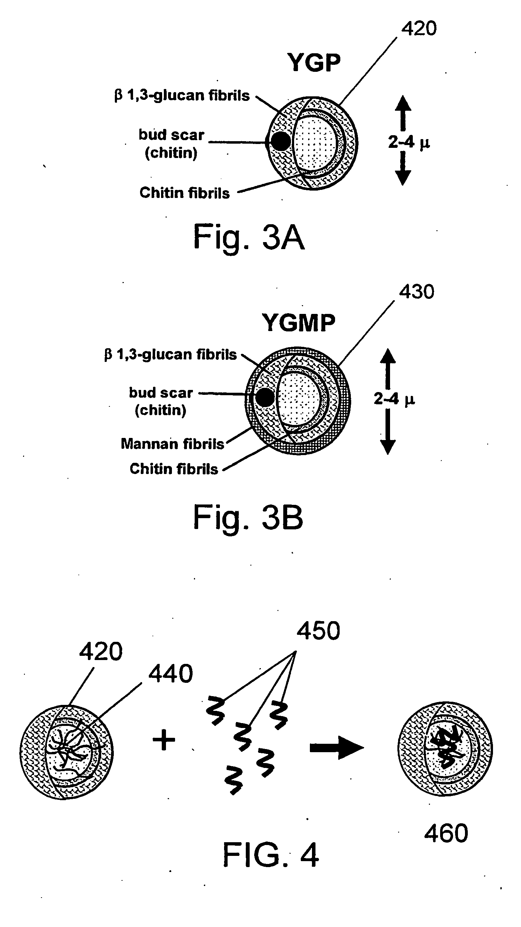

Fig. 3A is a schematic diagram of the structure of a YGP beta glucan particle 420, showing beta 1;3-glucan fibrils, the bud scar, which includes chitin, and and chitin fibrils. Fig. 3B is a schematic diagram of the structure of a YGMP beta glucan-mannan particle particle 430, showing beta 1,3-glucan fibrils, the bud scar, which includes chitin, mannan fibrils and chitin fibrils.
Fig. 4 is a schematic of an embodiment of the present invention, illustrating the process of loading a YGP particle 420 containing a trapping polymer 440 with a payload molecule 450, such as DNA, to form a delivery system YGP 460.

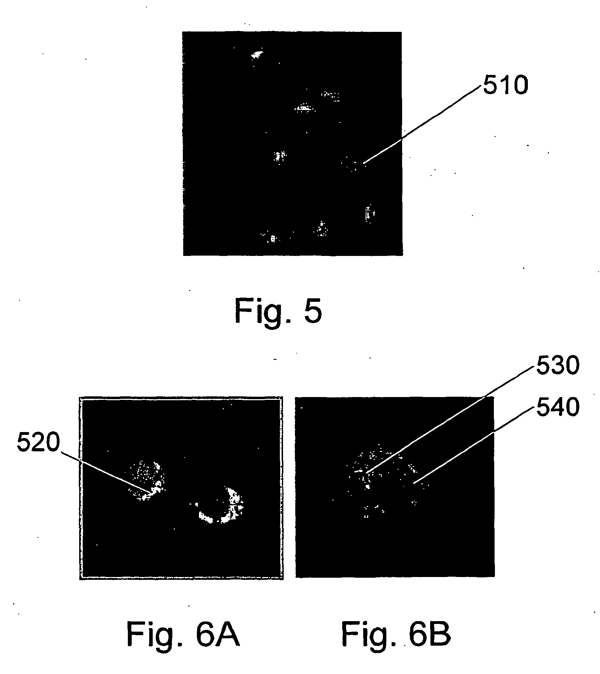

Fig. 5 is an image of a color fluorescence photomicrograph of J774 cells, e.g., an indicated cell 510 that had been exposed to YGP particles containing pIRES-EGFP, an expression vector encoding enhanced green fluorescent protein, a cationic trapping polymer PEI and cationic detergent CTAB, showing evidence of particle uptake and expression of the enhanced green fluorescent protein.
Fig. 6A and Fig. 6B are images of color fluorescence photomicrographs of bone marrow macrophages showing uptake of YGP-FITC particles 520 (Fig. 6A) and in Fig. 6B, uptake of YGP-FITC particles 530 and staining specific for the macrophage marker F4/80 540.

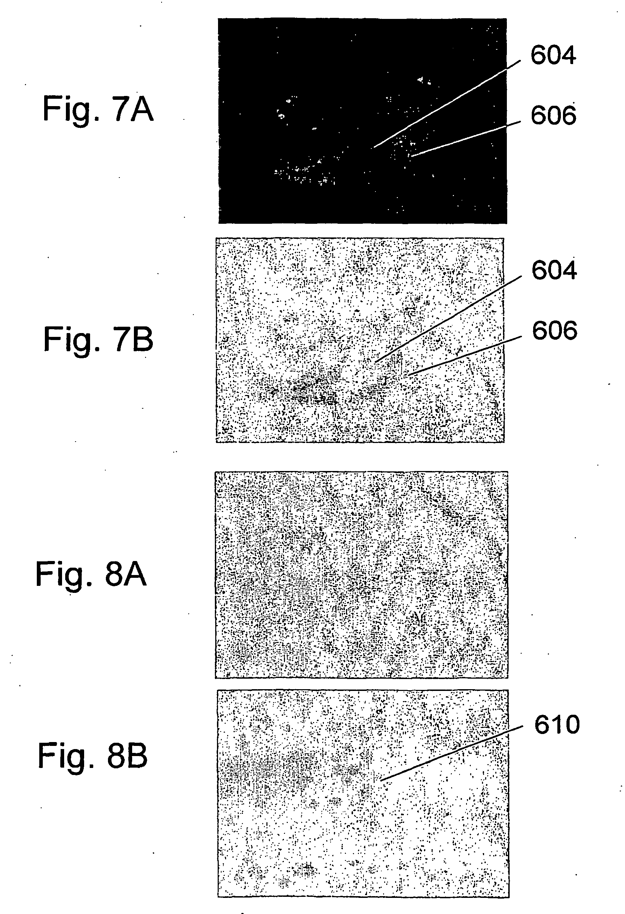

Fig. 7A is an image of a color fluorescence photomicrograph of murine RAW cells showing uptake of Texas Red labeled YCWP particles 606 loaded with a construct that produced the expression of green fluorescent protein (diffuse fluorescence 604. Fig. 7B is a contrast-reversed (negative) grayscale images of Fig. 7A.
Fig. 8A and Fig. 8B are images of color fluorescence photomicrographs of J774 cells sham transfected (Fig. 8A) or treated *in vitro* with YGP: pIRES2DsRED2-OPG (Fig. 8B). Human osteoprotegerin expression was detectable as immunoreactivity in >50% of J774 cells treated *in vitro* with YGP: pIRES2DsRED2-OPG formulations, such as indicated cell 610. The anti-human osteoprotegerin antibody selectively identified recombinant human osteoprotegerin and did not cross-react with endogenous mouse osteoprotegerin. These results demonstrate that YGP: pIRES2DsRED2-OPG formulations are effective in efficiently delivering the human osteoprotegerin encoding DNA, resulting in transient expression of human osteoprotegerin in murine J774 macrophage cells.

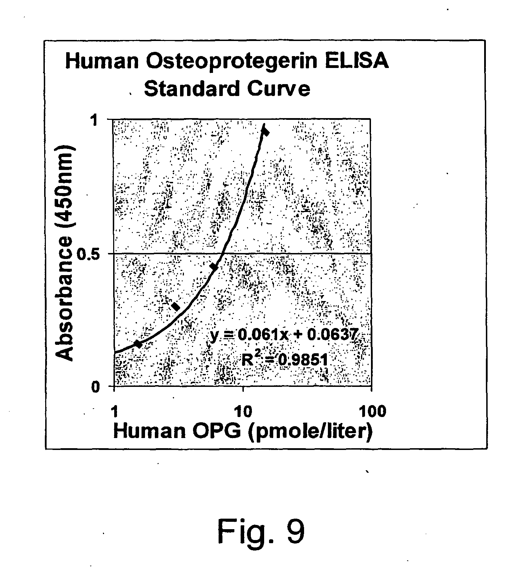

Fig. 9 is a graphical representation of a representative human osteoprotegerin ELISA standard curve.

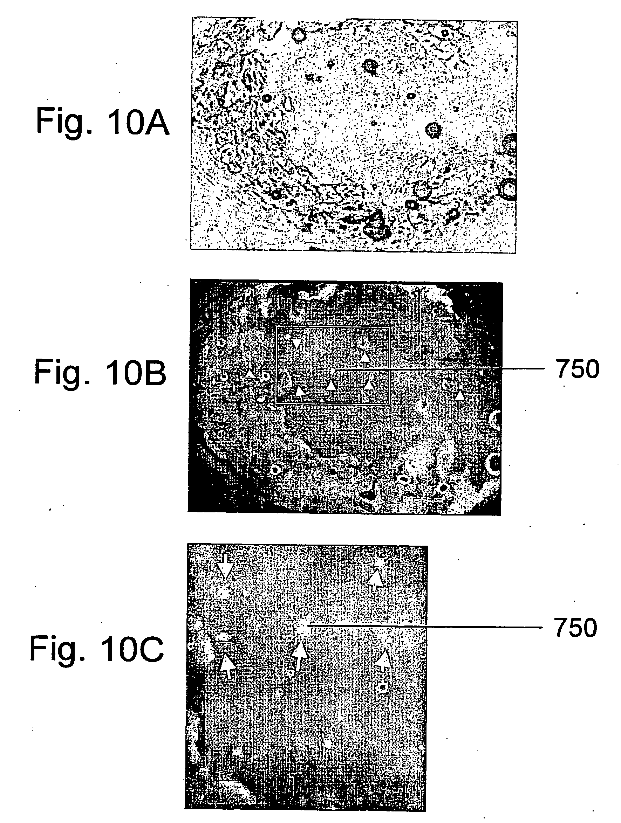

Fig. 10A - Fig. 10C show images of tissue sections of a femur from a mouse that had received an IP injection of fluorescently labeled YGP particles four days previously, showing that fluorescently labeled particles 750 were distributed to bone. Fig. 10A shows a bone section viewed under transmitted light. Fig. 10B shows the same field as in Fig. 10A viewed by fluorescence microscopy, showing several cells (arrows) that have fluorescently labeled particles 750. Fig. 10C is a higher magnification image that includes the field indicated by a rectangle in Fig. 10B.

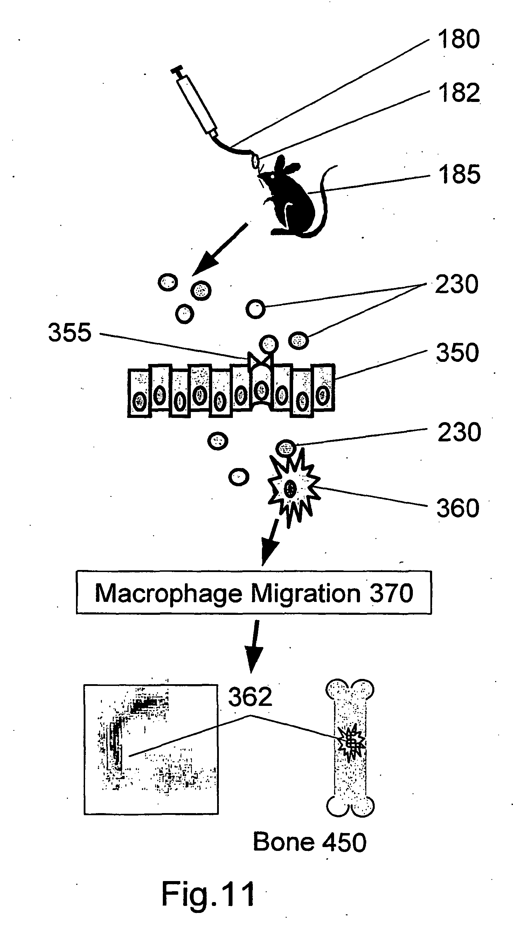

Fig. 11 is a schematic diagram of a preferred embodiment of the method of delivering yeast beta glucan particles (YGP) 230 by macrophage migration 370 to bone 450 after *in vivo* oral administration 180. A composition 182 containing yeast beta glucan particles (YGP) 230 is administered orally 180 to a subject 185. The yeast beta glucan particles (YGP) 230 are take up by M cells 355 in the lining of the small intestine and are translocated across the epithelium 350 and are phagocytosed by intestinal macrophages 360. The YGP-containing macrophages migrate 370 to various organs and tissues including bone 450. About 90 hours after oral administration, bone marrow macrophages 362 that had phagocytosed YGP were observed in bone 450 (shown both schematically and in a reversed contrast grayscale image of a color fluorescence photomicrograph).

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.

### DETAILED DESCRIPTION OF THE INVENTION

OPG Gene Therapy Using Yeast Cell Wall Particles as Delivery Vehicles. In preferred embodiments, die present invention provides compositions for the oral administration of micron-sized yeast cell wall particles containing DNA encoding human osteoprotegerin to overcome current limitations of therapy for low bone density and osteoporosis. In preferred embodiments, there is effective expression of osteoprotegerin in macrophages and osteoclasts in bone.

This delivery system is useful for *in vivo* or *in vitro* delivery of a wide range of payload molecules including, nucleic acids such as oligonucleotides, antisense constructs, siRNA, DNA constructs, including expression vectors, and peptides and proteins. The potential uses for this innovative macrophage-targeted delivery system are wide ranging based on the ability ofYCMP to deliver payloads that can up- and down-regulate macrophage gene expression combined with the use of macrophage trafficking to carry the orally administered payloads to sites of infection, inflammation, tumor or other pathology.

The present invention provides a therapeutic delivery system comprising an extracted yeast cell wall comprising beta-glucan, a payload trapping molecule and a payload molecule, wherein the payload molecule and the payload trapping molecule are soluble in the same solvent system wherein the payload molecule supplements the function of the deficient anti-osteoclastogenic bone protein. A particularly preferred protein is an osteroprogenin. The invention further provides methods of making and use of the therapeutic delivery system.

Advantageously, the composition of the present invention inherently directly targets macrophages and in preferred embodiments, provides an anti-osteoclastogenic protein. A particularly preferred anti-osteoclastogenic protein is OPG. Administering the therapeutic delivery system of the present invention by oral or mucosal or parenteral routes serves to avoid adverse effects of intravenous enzyme or protein replacement therapy. Supplementing the protein deficit by supplying an expression vector instead of the encoded protein itself serves to minimize or avoid antigenic reactions.

Advantagously, by targeting macrophages and other phagocytic cells, the present invention provides a means of delivering the therapeutic system to a diverse range of locations such as bone, kidney, lung, gastrointestinal tract and brain. While not being held to a particular theory, it is believed that the migration of macrophages and other phagocytic cells to a site is determined in part by one or more stimuli, such as inflammation, lipid, or other physiological macrophage attractants. Under this model, it is believed that the population of phagocytic cells bearing the therapeutic delivery system of the present invention in any particular, tissue is in dynamic equilibrium with similar populations in other tissues. Hence, the population of phagocytic cells bearing the therapeutic delivery system in any particular tissue, and thus the supplementation of the deficient protein, may fluctuate in time, responding, at least in part, to the physiological influences that act to regulate macrophage and other phagocytic cell distribution and activity.

In general, the compositions of the present invention provide simple, efficacious and efficient delivery of therapeutic agents *in vivo,* preferably by oral administration. The compositions have improved stability compared to available compositions, and have further advantages in patient convenience (and thus, patient compliance), lower costs and decreased or reduced side effects.

### Definitions

"Subject" means mammals and non-mammals. "Mammal" means any member of the class Mammalia including, but not limited to, humans, non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" does not denote a particular age or sex.

A "therapeutic effect" means an amelioration of the symptoms or reduction of progression of the disease; in osteoclastogenic control, "therapeutic effect" means a detectible increase in bone mass or bone density. A "therapeutically effective amount" means an amount of a compound that, when administered to a subject for treating a disease, is sufficient to cause such therapeutic effect. The "therapeutically effective amount" will vary depending on the compound, the disease state being treated, the severity or the disease treated, the age and relative health of the subject, the route and form of administration, the judgement of the attending medical or veterinary practitioner, and other factors. A "functional equivalent" of a protein means a molecule, protein or non-protein, that differs structurally from the protein but performs the same function as the protein under equivalent conditions. A "functional equivalent" of osteoprotegerin means a molecule, protein or non-protein, that differs structurally from the osteoprotegerin protein and acts to sequester RANKL under equivalent conditions. Osteoprotegerin is a member of the tumor necrosis factor receptor superfamily. Preferred functional equivalents of the osteoprotegerin protein include molecules including at least one tumor necrosis factor (TNFR) domain, such as a polypeptide consisting essentially of residues 28 to 124 of SEQ ID NO: 2, a polypeptide consisting essentially of residues 124 to 185 of SEQ ID NO: 2, and a polypeptide consisting essentially of residues 28 to 185 of SEQ ID NO: 2.

As used herein, "polyplexes" means polyelectrolyte complexes, especially polyelectrolyte complexes comprising a polynucleotide, such as plasmid DNA, and a polyionic polymer, such as cationic polymer. Preferred polyplexes of the present invention comprise a payload molecule that comprises an expression vector comprising a control element operatively linked to an open reading frame and a payload trapping molecule.

Payload Trapping Molecules. The payload trapping molecule is preferably a pharmaceutically acceptable excipient. The payload and trapping molecule are both soluble in the solvent system; the solvent system must be absorbed through the yeast cell particle carbohydrate matrix allowing the absorption of the payload and trapping polymer. The payload and trapping molecule are preferably water soluble. In preferred embodiments, the trapping molecule is biodegradable.

The mechanism of action of the trapping reaction with a given payload dictates the choice of payload trapping molecule. For electrostatic interactions a charged payload trapping molecule of opposite charge of the payload is required. For physical entrapment, the payload trapping molecule suitably participates in the formation of a matrix that reduces the diffusion of a payload. In other embodiments, the payload trapping molecule contributes a hydrophobic binding property that contributes to the retention of the payload. In further embodiments, the payload trapping molecule selectively binds to the payload, providing an affinity interaction that contributes to the retention of the payload.

In general, polyelectrolytes can be suitable payload trapping molecules. Several suitable polyelectrolytes are disclosed in U.S. Pat. No. 6,133,229. The polyelectrolyte may be a cationic or anionic polyelectrolyte. Amphoteric polyelectrolytes may also be employed. The cationic polyelectrolyte is preferably a polymer with cationic groups distributed along the molecular chain. The cationic groups, which in certain embodiments may include quaternary ammonium-derived moieties, may be disposed in side groups pendant from the chain or may be incorporated in it. Examples of cationic polyelectrolytes include: copolymers of vinyl pyrollidone and quaternary methyl methacrylate e.g., GAFQUAT®. series (755N, 734, HS-100) obtained from ISP; substituted polyacrylamides; polyethyleneimine, polypropyleneimine and substituted derivatives; polyamine homopolymers (GOLCHEM® CL118); polyamine co-polymers (e.g., condensates of epichlorohydrin and mono or dimethylamine); polydiallyl dimethyl ammonium chloride (polyDADMAC); substituted dextrans; modified guar gum (substituted with hydroxypropytrimonium chloride); substituted proteins (e.g., quaternary groups substituted on soya protein and hydrolysed collagen); polyamino acids (e.g., polylysine); low molecular weight polyamino compounds (e.g., spermine and spermidine). Natural or artificial polymers may be employed. Cationic polyelectrolytes with MW 150 to 5,000,000, preferably 5000 to 500,000, more preferably 5000 to 100,000 may be employed. An amount of 0.01 to 10% is preferred, more preferably 0.1 to 2% w/v, especially 0.05 to 5%.

The anionic polyelectrolyte is preferably a polymer with anionic groups distributed along the molecular chain. The anionic groups, which may include carboxylate, sulfonate, sulphate or other negatively charged ionisable grpupings, may be disposed upon groups pendant from the chain or bonded directly to the polymer backbone. Natural or artificial polymers may be employed.

Examples of anionic polyelectrolytes include: a copolymer of methyl vinyl ether and maleic anhydride, a copolymer of methyl vinyl ether and maleic acid, (Gantrez AN-series and S-series, respectively, International Specialty Products, Wayne, NJ); alginic acid and salts; carboxymethyl celluloses and salts; substituted polyacrylamides (eg substituted with carboxylic acid groups); polyacrylic acids and salts; polystyrene sulfonic acids and salts; dextran sulphates; substituted saccharides e.g., sucrose octosulfate; heparin. Anionic polyelectrolytes with MW of 150 to 5,000,000 may be used, preferably 5000 to 500,000, more preferably 5000 to 100,000. An amount of 0.01% to 10% is preferred especially 0.05 to 5% more especially 0.1 to 2% w/v.

Biological polymers, such as polysaccharides, are trapping polymers. Preferably, the polymers are processed to an average molecular weight to less than 100,000 Daltons. The polymers are preferably derivatized to provide cationic or anionic characteristics. Suitable polysaccharides include chitosan (deacetylated chitin), alginates, dextrans, such as 2-(diethylamino) ethyl ether dextran (DEAE-dextran) and dextran sulphate, xanthans, locust bean gums and guar gums (not all claimed).

Two general classes of cationic molecules are suitable for use as trapping molecules with negatively charged payloads such as nucleic acids: cationic polymers and cationic lipids.

A wide variety of cationic polymers have been shown to mediate *in vitro* transfection, ranging from proteins [such as histones (Fritz, J. D., et al, (1996) Hum. Gene Ther. 7, 1395-1404) and high mobility group (HMG) proteins (Mistry, A. R., et al. (1997) BioTechniques 22, 718-729)] and polypeptides [such as polylysine (Wu, G. Y. & Wu, C. H. (1987) J. Biol. Chem. 262, 4429-4432, Wagner, E., et al., (1991) Bioconjugate Chem. 2, 226-231,, short synthetic peptides (Gottschalk, S.,et al., (1996) Gene Ther. 3, 448-457; Wadhwa, M. S., et al., (1997) Bioconjugate Chem. 8, 81-88), and helical amphiphilic peptides (Legendre, J. Y., et al., (1997) Bioconjugate Chem. 8, 57-63; Wyman, T. B., et al., (1997) Biochemistry 36, 3008-3017)] to synthetic polymers [such as polyethyleneimine (Boussif, O., et al., (1996) Gene Ther. 3, 1074-1080), cationic dendrimers (Tang, M. X., et al., (1996) Bioconjugate Chem. 7, 703-714; Haensler, J. et al., (1993) Bioconjugate Chem. 4, 372-379), and glucaramide polymers (Goldman, C. K., et al., (1997) Nat. Biotech. 15,462-466)]. Other suitable cationic polymers include N-substituted glycine oligomers (peptoids) (Murphy, J.E., et al, A combinatorial approach to the discovery of efficient cationic peptoid reagents for gene delivery, Proc Natl Acad Sci. USA, 1998 95 (4)1517-1522), poly(2-methyl-acrylic acid 2-[(2-dimethylamino)-ethyl)-methyl-amino]-ethyl ester), abbreviated as pDAMA, and poly(2-dimethylamino ethyl)- methacrylate (pDMAEMA) (Funhoff, A.M., et al., 2004 Biomacromolecules, 5, 32-39).

Cationic lipids are also known in the art to be suitable for transfection. Felgner, P.L1, et al., Lipofection: a highly efficient, lipid-mediated DNA-transfection procedure.Proc Natl Acad Sci USA. 1987 84(21):7413-7. Suitable cationic lipids include N-[1-(2,3-dioleyloxy)propyl]-N,N,N-trimethylammonium chloride (DOTMA), [N,N,N',N'-tetramethyl-N,N'-bis(2-hydroxyethyl)-2,3-di(oleoyloxy)-1,4-butanediammonium iodide] (Promega Madison, WI, USA), dioctadecylamidoglycyl spermine (Promega Madison, WI, USA), N-[1-(2,3-Dioleoyloxy)]-N,N,N-trimethylammonium propane methylsulfate (DOTAP), N-[1-(2,3-dioleyloxy)propyl]-N,N,N-trimethylammonium chloride, 1,2-dimyristyloxypropyl-3-dimethyl-hydroxy ethyl ammonium bromide (DMRIE), dimyristoleoyl phosphonomethyl trimethyl ammonium (DMPTA) ( see Floch et al. 1997. Cationic phosphonolipids as non-viral vectors for DNA transfection in hematopoietic cell lines and CD34+ cells. Blood Cells, Molec.& Diseases 23: 69-87), 1,2-dioleoyl-sn-glycero-3-phosphoethanolamine-N-(7-nitro-2-1,3-benzoxadiazol-4-yl), ammonium salt (Avanti Polar Lipids, Inc. Alabaster, AL, US), 1,2-dioleoyl-3-trimethylammonium-propane chloride (Avanti Polar Lipids, Inc. Alabaster, AL, US), 1,2-dioleoyl-sn-glycero-3-phosphoethanolamine (Avanti Polar Lipids, Inc. Alabaster, AL, US) and 1,3-dioleoyloxy-2-(6-carboxyspermyl) propylamide (DOSPER).

Polyamines suitable as cationic trapping molecules are described in U.S. Pat. Nos. 6,379,965 and 6,372,499.

Payload Molecules. The particulate delivery system of the present invention is useful for *in vivo* or *in vitro* delivery of payload molecules including, but limited to, nucleic acids such as oligonucleotides, and recombinant DNA constructs, including expression vectors.

The particulate delivery system of the present invention may also be useful for *in vivo* or *in vitro* delivery of payload molecules such as amino acids, peptides and proteins. By "protein" is meant a sequence of amino acids for which the chain length is sufficient to produce the higher levels of tertiary and/or quaternary structure. This is to distinguish from "peptides" or other small molecular weight drugs that do not have such structure. Typically, the protein herein will have a molecular weight of at least about 15-20 kD, preferably at least about 20 kD.

Examples of proteins encompassed within the definition herein include mammalian proteins, such as, e.g., osteoprotegerin, growth hormone (GH), including human growth hormone, bovine growth hormone, and other members of the GH supergene family; growth hormone releasing factor, parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VIIIC, factor IX tissue factor, and von Willebrands factor; anti-clotting factors such as Protein C; atrial natriuretic factor, lung surfactant; a plasminogen activator, such as urokinase or tissue-type plasminogen activator (t-PA); bombazine; thrombin; alpha tumor necrosis factor, beta tumor necrosis factor, enkephalinase; RANTES (regulated on activation normally T-cell expressed and secreted); human macrophage inflammatory protein (MIP-1-alpha); serum albumin such as human serum albumin; mullerian-inhibiting substance; relaxin A-chain; relaxin B-chain; prorelaxin; mouse gonadotropin-associated peptide; DNase; inhibin; activin; vascular endothelial growth factor (VEGF); receptors for hormones or growth factors; an integrin; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-beta; platelet-derived growth factor (PDGF); fibroblast growth factor such as aFGF and bFGF; epidermal growth factor (EGF); transforming growth factor (TGF) such as TGF-alpha and TGF-beta, including TGF-beta1, TGF-beta2, TGF-beta3, TGF-beta4, or TGF-beta5; insulin-like growth factor-I and -II (IGF-I and IGF-II); des(1-3)-IGF-I (brain IGF-D; insulin-like growth factor binding proteins; CD proteins such as CD3, CD4, CD8, CD19 and CD20; osteoinductive factors; immunotoxins; a bone morphogenetic protein (BMP); T-cell receptors; surface membrane proteins; decay accelerating factor (DAF); a viral antigen such as, for example, a portion of the AIDS envelope; transport proteins; homing receptors; addressins; regulatory proteins; immunoadhesins; antibodies; and biologically active fragments or variants of any of the above-listed polypeptides. The protein may be osteoprotegerin or a functional equivalent thereof.

The members of the GH supergene family include growth hormone, prolactin, placental lactogen, erythropoietin, thrombopoietin, interleukin-2, interleukin-3, interleukin-4, interleukin-5, interleukin-6, interleukin-7, interleukin-9, interleukin-10, interleukin-11, interleukin-12 (p35 subunit), interleukin-13, interleukin-15, oncostatin M, ciliary neurotrophic factor, leukemia inhibitory factor, alpha interferon, beta interferon, gamma interferon, omega interferon, tau interferon, granulocyte-colony stimulating factor, granulocyte-macrophage colony stimulating factor, macrophage colony stimulating factor, cardiotrophin-1 and other proteins identified and classified as members of the family.

The protein payload molecule is preferably essentially pure and desirably essentially homogeneous (i.e. free from contaminating proteins etc). "Essentially pure" protein means a composition comprising at least about 90% by weight of the protein, based on total weight of the composition, preferably at least about 95% by weight. "Essentially homogeneous" protein means a composition comprising at least about 99% by weight of protein, based on total weight of the composition. Proteins may be derived from naturally occurring sources or produced by recombinant technology. Proteins include protein variants produced by amino acid substitutions or by directed protein evolution (Kurtzman, A.L., et al., Advances in directed protein evolution by recursive genetic recombination: applications to therapeutic proteins, Curr Opin Biotechnol. 2001 12(4): 361-70) as well as derivatives, such as PEGylated proteins.

Antibodies. The protein payload molecules may also be an antibody (not claimed). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. Thus, these fragments are preferred, as well as the products of a Fab or other immunoglobulin expression library. Moreover, antibodies of the present invention include chimeric, single chain, and humanized antibodies.

Antibodies can be prepared using any number of techniques known in the art Suitable techniques are discussed briefly below. The antibody may be polyclonal or monoclonal. Polyclonal antibodies can have significant advantages for initial development, including rapidity of production and specificity for multiple epitopes, ensuring strong immunofluorescent staining and antigen capture. Monoclonal antibodies are adaptable to large-scale production; preferred embodiments include at least one monoclonal antibody specific for an epitope of the target antigen. Because polyclonal preparations cannot be readily reproduced for large-scale production, another embodiment uses a cocktail of at least four monoclonal antibodies.

A single chain Fv ("scFv" or "sFv") polypeptide is a covalently linked V_{H} :V_{L} heterodimer which may be expressed from a nucleic acid including V_{H} - and V_{L} - encoding sequences either joined directly or joined by a peptide-encoding linker. Huston, et al. Proc. Nat. Acad. Sci. USA, 85: 5879-5883 (1988). A number of structures for converting the naturally aggregated, but chemically separated, light and heavy polypeptide chains from an antibody V region into a scFv molecule which folds into a three dimensional structure substantially similar to the structure of an antigen-binding site. See,.e.g., U.S. Pat. Nos. 6,512,097, 5,091,513 and 5,132,405 and 4,956,778.

In one class of embodiments, recombinant design methods can be used to develop suitable chemical structures (linkers) for converting two naturally associated, but chemically separate, heavy and light polypeptide chains from an antibody variable region into a sFv molecule which folds into a three-dimensional structure that is substantially similar to native antibody structure. Design criteria include determination of the appropriate length to span the distance between the C-terminal of one chain and the N-terminal of the other, wherein the linker is generally formed from small hydrophilic amino acid residues that do not tend to coil or form secondary structures. Such methods have been described in the art. See, e.g., U.S. Pat. Nos. 5,091,513 and 5,132,405 to Huston et al.; and U.S. Pat. No. 4,946,778 to Ladner et al.

In this regard, the first general step of linker design involves identification of plausible sites to be linked. Appropriate linkage sites on each of the V_{H} and V_{L} polypeptide domains include those which result in the minimum loss of residues from the polypeptide domains, and which necessitate a linker comprising a minimum number of residues consistent with the need for molecule stability. A pair of sites defines a "gap" to be linked. Linkers connecting the C-terminus of one domain to the N-terminus of the next generally comprise hydrophilic amino acids which assume an unstructured configuration in physiological solutions and preferably are free of residues having large side groups which might interfere with proper folding of the V_{H} and V_{L} chains. Thus, suitable linkers under the invention generally comprise polypeptide chains of alternating sets of glycine and serine residues, and may include glutamic acid and lysine residues inserted to enhance solubility. Nucleotide sequences encoding such linker moieties can be readily provided using various oligonucleotide synthesis techniques known in the art.

Alternatively, a humanized antibody fragment may comprise the antigen binding site of a murine monoclonal antibody and a variable region fragment (lacking the antigen binding site) derived from a human antibody. Procedures for the production of chimeric and further engineered monoclonal antibodies include those described in Riechmann et al. (Nature 332 : 323,1988), Liu et al. (PNAS 84 : 3439,1987), Larrick et al. (Bio Technology 7: 934,1989), and Winter and Harris (TIPS 14 : 139, May, 1993).

One method for producing a human antibody comprises immunizing a nonhuman animal, such as a transgenic mouse, with a target antigen, whereby antibodies directed against the target antigen are generated in said animal. Procedures have been developed for generating human antibodies in non-human animals. The antibodies may be partially human, or preferably completely human Non-human animals (such as transgenic mice) into which genetic material encoding one or more human immunoglobulin chains has been introduced may be employed. Such transgenic mice may be genetically altered in a variety of ways. The genetic manipulation may result in human immunoglobulin polypeptide chains replacing endogenous immunoglobulin chains in at least some (preferably virtually all) antibodies produced by the animal upon immunization, Antibodies produced by immunizing transgenic animals with a target antigen are provided herein.

Mice in which one or more endogenous immunoglobulin genes are inactivated by various means have been prepared. Human immunoglobulin genes have been introduced into me mice to replace the inactivated mouse genes. Antibodies produced in the animals incorporate human immunoglobulin polypeptide chains encoded by the human genetic material introduced into the animal. Examples of techniques for production and use of such transgenic animals are described in U. S. Patents 5,814,318, 5,569,825, and 5,545,806.

Monoclonal antibodies may be produced by conventional procedures, e. g., by immortalizing spleen cells harvested from the transgenic animal after completion of the immunization schedule. The spleen cells may be fused with myeloma cells to produce hybridomas, by conventional procedures.

A method for producing a hybridoma cell line comprises immunizing such a transgenic animal with a immunogen comprising at least seven contiguous amino acid residues of a target antigen; harvesting spleen cells from the immunized animal; fusing the harvested spleen cells to a myeloma cell line, thereby generating hybridoma cells; and identifying a hybridoma cell line that produces a monoclonal antibody that binds a target antigen. Such hybridoma cell lines, and monoclonal antibodies produced therefrom, are encompassed by the present invention. Monoclonal antibodies secreted by the hybridoma cell line are purified by conventional techniques.

In another possibility antibody fragments are produced by selection from a nonimmune phage display antibody repertoire against one set of antigens in the presence of a competing set of antigens (Stausbol-Grøn, B., et al., De novo identification of cell-type specific antibody-antigen pairs by phage display subtraction. Isolation of a human single chain antibody fragment against human keratin 14. Eur J Biochem 2001 May; 268(10):3099-107). This approach can be used to produce phage antibodies directed against target antigens. The protocol in general is based on that described by Stausbol-Grøn, B., et al., 2001. Briefly, a nonimmunized semisynthetic phage display antibody repertoire is used. The repertoire is a single chain Fv (scFv) phagemid repertoire constructed by recloning the heavy and light chain regions from the lox library (Griffiths, A.D., et al. (1994) Isolation of high affinity human antibodies directly from large synthetic repertoires. EMBO J. 13, 3245-3260.). *Eseheriehia coli* TG1 (*supE* hsdD5 Δ(*lac-proAB*) *thi* F'{*traD*36 *pro*AB+ *lacl*^{q} *lac*ZΔM15]) is an amber suppressor strain *(supE)* and is used for propagation of phage particles. *E. coli* HB2151 (*ara Δ(lac-proAB) thi* F'{*pro*AB+ *lacl*^{q} *lac*ZΔM15]) is a nonsuppressor strain and is used for expression of soluble scFv. In another embodiment, a human single-chain Fv (scFv) library can be amplified and rescued, as described (Gao, at al., Making chemistry selectable by linking it to infectivity, Proc. Natl. Acad. Sci. USA, Vol. 94, pp. 11777-11782, October 1997). The library is panned against target antigens suspended in PBS (10 mM phosphate, 150 mM NaCl, pH 7.4) and the positive scFv-phage are selected by enzyme-linked immunosorbent assay (ELISA).

An antibody may be supplied by providing an expression vector encoding a recombinant antibody, preferably a single chain Fv antibody.

Gene Therapy. The Human Genome Project has increased our knowledge of the genetic basis of disease. See, generally, http://www.ornl.gov/sci/techresources/ Human Genome/medicine/assist.shtml. In preferred embodiments, the present invention provides compositions and methods for the treatment of genetic disorders or conditions having a genetic component. In further preferred embodiments, the present invention provides compositions useful for the manufacture of pharmaceutical products for the treatment of genetic disorders or conditions having a genetic component.

In preferred embodiments, the particulate delivery system of the present invention is used to administer at least one nucleic acid comprising a compensating gene. In other preferred embodiments, the particulate delivery system of the present invention is used to administer at least one nucleic acid encoding a gene product of a missing gene, wherein the expression of the gene product is useful in the treatment of the genetic disorder or the genetic component of a condition. In preferred embodiments, the particulate delivery system of the present invention including the desired payload molecule is useful for the manufacture of a pharmaceutical product for the treatment of genetic disorder or the genetic component of a condition. Such pharmaceutical products are suitably administered orally, rectally, parenterally, (for example, intravenously, intramuscularly, or subcutaneously) intracisternally, intravaginally, intraperitoneally, intravesically, locally (for example, powders, ointments or drops), or as a buccal or nasal spray. The pharmaceutical products are preferably administered orally, buccally, and parenterally, more preferably orally. Particles loaded with different payloads, e.g., a nucleic acid, a nucleic acid expression vector or a small molecule therapeutic can be mixed in the appropriate proportions and administered together, e.g., in a capsule, for combination therapy.

In aspects of the present invention that relate to gene therapy, the nucleic acid compositions contain either compensating genes or genes that encode therapeutic proteins. Examples of genes encoding therapeutic proteins include genes which encode osteoprotegerin . In preferred embodiments, the protein is osteoprotegerin or a functional equivalent thereof

Routes of Administration. Routes of administration include but are not limited to oral, buccal, sublingual, pulmonary, transdermal, transmucosal, as well as subcutaneous, intraperitoneal, intravenous, and intramuscular injection. Preferred routes of administration are oral, buccal, sublingual, pulmonary and transmucosal.

The particulate delivery system of the present invention is administered to a subject in a therapeutically effective amount. The particulate delivery system can be administered alone or as part of a pharmaceutically acceptable composition. In addition, a compound or composition can be administered all at once, as for example, by a bolus injection, multiple times, such as by a series of tablets, or delivered substantially uniformly over a period of time, as for example, using a controlled release formulation. It is also noted that the dose of the compound can be varied over time. The particulate delivery system can be administered using an immediate release formulation, a controlled release formulation, or combinations thereof. The term "controlled release" includes sustained release, delayed release, and combinations thereof.

A pharmaceutical composition of the invention can be prepared, packaged, or sold in bulk, as a single unit dose, or as a plurality of single unit doses. As used herein, a "unit dose" is discrete amount of the pharmaceutical composition comprising a predetermined amount of the active ingredient. The amount of the active ingredient is generally equal to the dosage of the active ingredient that would be administered to a subject or a convenient fraction of such a dosage such as, for example, one-half or one-third of such a dosage.

The relative amounts of the active ingredient, the pharmaceutically acceptable carrier, and any additional ingredients in a pharmaceutical composition of the invention will vary, depending upon the identity, size, and condition of the human treated and further depending upon the route by which the composition is to be administered. By way of example, the composition can comprise between 0.1% and 100% (w/w) active ingredient. A unit dose of a pharmaceutical composition of the invention will generally comprise from about 100 milligrams to about 2 grams of the active ingredient, and preferably comprises from about 200 milligrams to about 1.0 gram of the active ingredient.

In addition, a particulate delivery system of the present invention can be administered alone, in combination with a particulate delivery system with a different payload, or with other pharmaceutically active compounds. The other pharmaceutically active compounds can be selected to treat the same condition as the particulate delivery system or a different condition.

If the subject is to receive or is receiving multiple pharmaceutically active compounds, the compounds can be administered simultaneously or sequentially in any order. For example, in the case of tablets, the active compounds may be found in one tablet or in separate tablets, which can be administered at once or sequentially in any order. In addition, it should be recognized that the compositions can be different forms. For example, one or more compounds may be delivered via a tablet, while another is administered via injection or orally as a syrup.

Another aspect of the invention relates to a kit comprising a pharmaceutical composition of the invention and instructional material. Instructional material includes a publication, a recording, a diagram, or any other medium of expression which is used to communicate the usefulness of the pharmaceutical composition of the invention for one of the purposes set forth herein in a human. The instructional material can also, for example, describe an appropriate dose of the pharmaceutical composition of the invention. The instructional material of the kit of the invention can, for example, be affixed to a container which contains a pharmaceutical composition of the invention or be shipped together with a container which contains the pharmaceutical composition. Alternatively, the instructional material can be shipped separately from the container with the intention that the instructional material and the pharmaceutical composition be used cooperatively by the recipient.

The invention also includes a kit comprising a pharmaceutical composition of the invention and a delivery device for delivering the composition to a human. By way of example, the delivery device can be a squeezable spray bottle, a metered-dose spray bottle, an aerosol spray device, an atomizer, a dry powder delivery device, a self-propelling solvent/powder-dispensing device, a syringe, a needle, a tampon, or a dosage- measuring container. The kit can further comprise an instructional material as described herein.

For example, a kit may comprise two separate pharmaceutical compositions comprising respectively a first composition comprising a particulate delivery system and a pharmaceutically acceptable carrier; and composition comprising second pharmaceutically active compound and a pharmaceutically acceptable carrier. The kit also comprises a container for the separate compositions, such as a divided bottle or a divided foil packet. Additional examples of containers include syringes, boxes, bags, and the like. Typically, a kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of a kit is a blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and a sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen that the tablets or capsules so specified should be ingested. Another example of such a memory aid is a calendar printed on the card, e.g., as follows "First Week, Monday, Tuesday, ... etc.... Second Week, Monday, Tuesday," etc. Other variations of memory aids will be readily apparent. A "daily dose" can be a single tablet or capsule or several pills or capsules to be taken on a given day. Also, a daily dose of a particulate delivery system composition can consist of one tablet or capsule, while a daily dose of the second compound can consist of several tablets or capsules and vice versa. The memory aid should reflect this and assist in correct administration.

In another embodiment of the present invention, a dispenser designed to dispense the daily doses one at a time in the order of their intended use is provided. Preferably, the dispenser is equipped with a memory aid, so as to further facilitate compliance with the dosage regimen. An example of such a memory aid is a mechanical counter, which indicates the number of daily doses that have been dispensed. Another example of such a memory aid is a battery-powered micro-chip memory coupled with a liquid crystal readout, or audible reminder signal which, for example, reads out the date that the last daily dose has been taken and/or reminds one when the next dose is to be taken.

A particulate delivery system composition, optionally comprising other pharmaceutically active compounds, can be administered to a subject either orally, rectally, parenterally, (for example, intravenously, intramuscularly, or subcutaneously) intracisternally, intravaginally, intraperitoneally, intravesically, locally (for example, powders, ointments or drops), or as a buccal or nasal spray.

Parenteral administration of a pharmaceutical composition includes any route of administration characterized by physical breaching of a tissue of a human and administration of the pharmaceutical composition through the breach in the tissue. Parenteral administration thus includes administration of a pharmaceutical composition by injection of the composition, by application of the composition through a surgical incision, by application of the composition through a tissue-penetrating non-surgical wound, and the like. In particular, parenteral administration includes subcutaneous, intraperitoneal, intravenous, intraarterial, intramuscular, or intrasternal injection and intravenous, intraarterial, or kidney dialytic infusion techniques.

Compositions suitable for parenteral injection comprise the active ingredient combined with a pharmaceutically acceptable carrier such as physiologically acceptable sterile aqueous or nonaqueous solutions, dispersions, suspensions, or emulsions, or may comprise sterile powders for reconstitution into sterile injectable solutions or dispersions. Examples of suitable aqueous and nonaqueous carriers, diluents, solvents, or vehicles include water, isotonic saline, ethanol, polyols (propylene glycol, polyethylene glycol, glycerol, and the like), suitable mixtures thereof, triglycerides, including vegetable oils such as olive oil, or injectable organic esters such as ethyl oleate. Proper fluidity can be maintained, for example, by the use of a coating such as lecithin, by the maintenance of the required particle size in the case of dispersions, and/or by the use of surfactants. Such formulations can be prepared, packaged, or sold in a form suitable for bolus administration or for continuous administration. Injectable formulations can be prepared, packaged, or sold in unit dosage form, such as in ampules, in multi-dose containers containing a preservative, or in single-use devices for auto-injection or injection by a medical practitioner.

Formulations for parenteral administration include suspensions, solutions, emulsions in oily or aqueous vehicles, pastes, and implantable sustained-release or biodegradable formulations. Such formulations can further comprise one or more additional ingredients including suspending, stabilizing, or dispersing agents. In one embodiment of a formulation for parenteral administration, the active ingredient is provided in dry (i.e. powder or granular) form for reconstitution with a suitable vehicle (e.g., sterile pyrogen-free water) prior to parenteral administration of the reconstituted composition. The pharmaceutical compositions can be prepared, packaged, or sold in the form of a sterile injectable aqueous or oily suspension or solution. This suspension or solution can be formulated according to the known art, and can comprise, in addition to the active ingredient, additional ingredients such as the dispersing agents, wetting agents, or suspending agents described herein. Such sterile injectable formulations can be prepared using a non-toxic parenterally-acceptable diluent or solvent, such as water or 1,3-butanediol, for example. Other acceptable diluents and solvents include Ringers solution, isotonic sodium chloride solution, and fixed oils such as synthetic mono- or di-glycerides. Other parentally-administrable formulations which are useful include those which comprise the active ingredient in microcrystalline form, in a liposomal preparation, or as a component of a biodegradable polymer system. Compositions for sustained release or implantation can comprise pharmaceutically acceptable polymeric or hydrophobic materials such as an emulsion, an ion exchange resin, a sparingly soluble polymer, or a sparingly soluble salt.

These compositions may also contain adjuvants, such as preserving, wetting, emulsifying, and/or dispersing agents. Prevention of microorganism contamination of the compositions can be accomplished by the addition of various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents capable of delaying absorption, for example, aluminum monostearate and/or gelatin.

Dosage forms can include solid or injectable implants or depots. In preferred embodiments, the implant comprises an aliquot of the particulate delivery system and a biodegradable polymer. In preferred embodiments, a suitable biodegradable polymer can be selected from the group consisting of a polyaspartate, polyglutamate, poly(L-lactide), a poly(D,L-lactide), a poly(lactide-co-glycolide), a poly(ε-caprolactone), a polyanhydride, a poly(beta-hydroxy butyrate), a poly(ortho ester) and a polyphosphazene.

Solid dosage forms for oral administration include capsules, tablets, powders, and granules. In such solid dosage forms, the particulate delivery system is optionally admixed with at least one inert customary excipient (or carrier) such as sodium citrate or dicalcium phosphate or (a) fillers or extenders, as for example, starches, lactose, sucrose, mannitol, or silicic acid; (b) binders, as for example, carboxymethylcellulose, alginates, gelatin, polyvinylpyrrolidone, sucrose, or acacia; (c) humectants, as for example, glycerol; (d) disintegrating agents, as for example, agar-agar, calcium carbonate, potato or tapioca starch, alginic acid, certain complex silicates, or sodium carbonate; (e) solution retarders, as for example, paraffin; (f) absorption accelerators, as for example, quaternary ammonium compounds; (g) wetting agents, as for example, cetyl alcohol or glycerol monostearate; (h) adsorbents, as for example, kaolin or bentonite; and/or (i) lubricants, as for example, talc, calcium stearate, magnesium stearate, solid polyethylene glycols, sodium lauryl sulfate, or mixtures thereof. In the case of capsules and tablets, the dosage forms may also comprise buffering agents.

A tablet comprising the particulate delivery system can, for example, be made by compressing or molding the active ingredient, optionally with one or more additional ingredients. Compressed tablets can be prepared by compressing, in a suitable device, the active ingredient in a free-flowing form such as a powder or granular preparation, optionally mixed with one or more of a binder, a lubricant, an excipient, a surface active agent, and a dispersing agent. Molded tablets can be made by molding, in a suitable device, a mixture of the active ingredient, a pharmaceutically acceptable carrier, and at least sufficient liquid to moisten the mixture. Pharmaceutically acceptable excipients used in the manufacture of tablets include inert diluents, granulating and disintegrating agents, binding agents, and lubricating agents. Known dispersing agents include potato starch and sodium starch glycolate. Known surface active agents include sodium lauryl sulfate. Known diluents include calcium carbonate, sodium carbonate, lactose, microcrystalline cellulose, calcium phosphate, calcium hydrogen phosphate, and sodium phosphate. Known granulating and disintegrating agents include com starch and alginic acid. Known binding agents include gelatin, acacia, pre-gelatinized maize starch, polyvinylpyrrolidone, and hydroxypropyl methylcellulose. Known lubricating agents include magnesium stearate, stearic acid, silica, and talc.

Tablets can be non-coated or they can be coated using known methods to achieve delayed disintegration in the gastrointestinal tract of a human, thereby providing sustained release and absorption of the particulate delivery system, e.g., in the region of the Peyer's patches in the small intestine. By way of example, a material such as glyceryl monostearate or glyceryl distearate can be used to coat tablets. Further by way of example, tablets can be coated using methods described in U.S. Pat. Nos. 4,256,108; 4,160,452; and 4,265,874 to form osmotically-controlled release tablets. Tablets can further comprise a sweetening agent, a flavoring agent, a coloring agent, a preservative, or some combination of these in order to provide pharmaceutically elegant and palatable preparation.

Solid dosage forms such as tablets, dragees, capsules, and granules can be prepared with coatings or shells, such as enteric coatings and others well known in the art. They may also contain opacifying agents, and can also be of such composition that they release the particulate delivery system in a delayed manner. Examples of embedding compositions that can be used are polymeric substances and waxes. The active compounds can also be in micro-encapsulated form, if appropriate, with one or more of the above-mentioned excipients.

Solid compositions of a similar type may also be used as fillers in soft or hard filled gelatin capsules using such excipients as lactose or milk sugar, as well as high molecular weight polyethylene glycols, and the like. Hard capsules comprising the particulate delivery system can be made using a physiologically degradable composition, such as gelatin. Such hard capsules comprise the particulate delivery system, and can further comprise additional ingredients including, for example, an inert solid diluent such as calcium carbonate, calcium phosphate, or kaolin. Soft gelatin capsules comprising the particulate delivery system can be made using a physiologically degradable composition, such as gelatin. Such soft capsules comprise the particulate delivery system, which can be mixed with water or an oil medium such as peanut oil, liquid paraffin, or olive oil.

Oral compositions can be made, using known technology, which specifically release orally-administered agents in the small or large intestines of a human subject. For example, formulations for delivery to the gastrointestinal system, including the colon, include enteric coated systems, based, e.g., on methacrylate copolymers such as poly(methacrylic acid, methyl methacrylate), which are only soluble at pH 6 and above, so that the polymer only begins to dissolve on entry into the small intestine. The site where such polymer formulations disintegrate is dependent on the rate of intestinal transit and the amount of polymer present. For example, a relatively thick polymer coating is used for delivery to the proximal colon (Hardy et al., 1987 Aliment. Pharmacol. Therap. 1:273-280). Polymers capable of providing site-specific colonic delivery can also be used, wherein the polymer relies on the bacterial flora of the large bowel to provide enzymatic degradation of the polymer coat and hence release of the drug. For example, azopolymers (U.S. Pat. No. 4,663,308), glycosides (Friend et al., 1984, J. Med. Chem. 27:261-268) and a variety of naturally available and modified polysaccharides (see PCT application PCT/GB89/00581) can be used in such formulations.

Pulsed release technology such as that described in U.S. Pat. No. 4,777,049 can also be used to administer the particulate delivery system to a specific location within the gastrointestinal tract. Such systems permit delivery at a predetermined time and can be used to deliver the particulate delivery system, optionally together with other additives that my alter the local microenvironment to promote stability and uptake, directly without relying on external conditions other than the presence of water to provide *in vivo* release.

Liquid dosage forms for oral administration include pharmaceutically acceptable emulsions, solutions, suspensions, syrups, and elixirs. In addition to the active compounds, the liquid dosage form may contain inert diluents commonly used in the art, such as water or other solvents, isotonic saline, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, almond oil, arachis oil, coconut oil, cottonseed oil, groundnut oil, com germ oil, olive oil, castor oil, sesame seed oil, MIGLYOL^{™}, glycerol, fractionated vegetable oils, mineral oils such as liquid paraffin, tetrahydrofurfuryl alcohol, polyethylene glycols, fatty acid esters of sorbitan, or mixtures of these substances, and the like. Besides such inert diluents, the composition can also include adjuvants, such as wetting agents, emulsifying and suspending agents, demulcents, preservatives, buffers, salts, sweetening, flavoring, coloring and perfuming agents. Suspensions, in addition to the active compound, may contain suspending agents, as for example, ethoxylated isostearyl alcohols, polyoxyethylene sorbitol or sorbitan esters, microcrystalline cellulose, hydrogenated edible fats, sodium alginate, polyvinylpyrrolidone, gum tragacanth, gum acacia, agar-agar, and cellulose derivatives such as sodium carboxymethylcellulose, methylcellulose; hydroxypropylmethylcellulose, aluminum metahydroxide, bentonite, or mixtures of these substances, and the like. Liquid formulations of a pharmaceutical composition of the invention that are suitable for oral administration can be prepared, packaged, and sold either in liquid form or in the form of a dry product intended for reconstitution with water or another suitable vehicle prior to use.

Known dispersing or wetting agents include naturally-occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (e.g., polyoxyethylene stearate, heptadecaethyleneoxycetanol, polyoxyethylene sorbitol monooleate, and polyoxyethylene sorbitan monooleate, respectively). Known emulsifying agents include lecithin and acacia. Known preservatives include methyl, ethyl, or n-propyl-para-hydroxybenzoates, ascorbic acid, and sorbic acid. Known sweetening agents include, for example, glycerol, propylene glycol, sorbitol, sucrose, and saccharin. Known thickening agents for oily suspensions include, for example, beeswax, hard paraffin, and cetyl alcohol.

In other embodiments, the pharmaceutical composition can be prepared as a nutraceutical, i.e., in the form of, or added to, a food (e.g., a processed item intended for direct consumption) or a foodstuff (e.g., an edible ingredient intended for incorporation into a food prior to ingestion). Examples of suitable foods include candies such as lollipops, baked goods such as crackers, breads, cookies, and snack cakes, whole, pureed, or mashed fruits and vegetables, beverages, and processed meat products. Examples of suitable foodstuffs include milled grains and sugars, spices and other seasonings, and syrups. The particulate delivery systems described herein are preferably not exposed to high cooking temperatures for extended periods of time, in order to minimize degradation of the compounds.

Compositions for rectal or vaginal administration can be prepared by mixing a particulate delivery system with suitable non-irritating excipients or carriers such as cocoa butter, polyethylene glycol or a suppository wax, which are solid at ordinary room temperature, but liquid at body temperature, and therefore, melt in the rectum or vaginal cavity and release the particulate delivery system. Such a composition can be in the form of, for example, a suppository, a retention enema preparation, and a solution for rectal or colonic irrigation. Suppository formulations can further comprise various additional ingredients including antioxidants and preservatives. Retention enema preparations, or solutions for rectal or colonic irrigation can be made by combining the active ingredient with a pharmaceutically acceptable liquid carrier. As is known in the art, enema preparations can be administered using, and can be packaged within, a delivery device adapted to the rectal anatomy of a human. Enema preparations can further comprise various additional ingredients including antioxidants and preservatives.

A pharmaceutical composition of the invention can be prepared, packaged, or sold in a formulation suitable for pulmonary administration via the buccal cavity. Such compositions are conveniently in the form of dry powders for administration using a device comprising a dry powder reservoir to which a stream of propellant can be directed to disperse the powder or using a self-propelling solvent/powder-dispensing container such as a device comprising the particulate delivery system suspended in a low-boiling propellant in a sealed container. Dry powder compositions may include a solid fine powder diluent such as sugar and are conveniently provided in a unit dose form. Low boiling propellants generally include liquid propellants having a boiling point below 65 degrees F. at atmospheric pressure. Generally the propellant can constitute 50 to 99.9% (w/w) of the composition, and the active ingredient can constitute 0.1 to 20% (w/w) of the composition. The propellant can further comprise additional ingredients such as a liquid non-ionic or solid anionic surfactant or a solid diluent (preferably having a particle size of the same order as particles comprising the particulate delivery system).

Pharmaceutical compositions of the invention formulated for pulmonary delivery can also provide the active ingredient in the form of droplets of a suspension. Such formulations can be prepared, packaged, or sold as aqueous or dilute alcoholic suspensions, optionally sterile, comprising the particulate delivery system, and can conveniently be administered using any nebulization or atomization device. Such formulations can further comprise one or more additional ingredients including a flavoring agent such as saccharin sodium, a volatile oil, a buffering agent, a surface active agent, or a preservative such as methylhydroxybenzoate.

The formulations described herein as being useful for pulmonary delivery are also useful for intranasal delivery of a pharmaceutical composition of the invention. Another formulation suitable for intranasal administration is a coarse powder comprising the particulate delivery system. Such a formulation is administered in the manner in which snuff is taken i.e. by rapid inhalation through the nasal passage from a container of the powder held close to the nares.

A pharmaceutical composition of the invention can be prepared, packaged, or sold in a formulation suitable for buccal administration. Such formulations can, for example, be in the form of tablets or lozenges made using conventional methods, and can, for example, comprise 0.1 to 20% (w/w) particulate delivery system, the balance comprising an orally dissolvable or degradable composition and, optionally, one or more of the additional ingredients described herein. Alternately, formulations suitable for buccal administration can comprise a powder or an aerosolized or atomized solution or suspension comprising the particulate delivery system.

### Animal Models for Evaluation of Therapy for Low Bone Density

The Osteopenic C57B1/6J Mouse. Variation in human populations leads to significant differences in peak bone mineral density and skeletal mass, and as much as 70% of these differences can be accounted for by genetic variation. Not surprisingly, there is an inverse correlation between peak bone mineral density and risk of osteoporosis. Bone mineral density, mechanical strength, and bone quality, parameters also vary significantly between different inbred strains of mice, as careful phenotypic comparisons of 11 such strains revealed (Turner, C. H., et al., (2001)Variation in bone biomechanical properties, microstructure, and density in BXH recombinant inbred mice. J Bone Miner Res 16, 206-213; Beamer, W. G., et al., (1996). Genetic variability in adult bone density among inbred strains of mice. Bone 18, 397-403.). The genetic basis for these differences has been investigated, and it has become evident that genetic control of skeletal growth and maintenance requires numerous genetic loci, and further, that bone mass at different skeletal sites such as the spine and limbs are influenced by different genetic factors. Overall, the lowest bone density, lowest trabecular bone volume fraction, and thinnest cortical bone among the strains investigated occurred in the C57B1/6J (B6) strain, and the highest were in the C3H/HEJ strain. B6 total femur bone mineral density was less than 66% of C3H/HEJ(C3H), whereas bone length and total body mass did not vary significantly . Further work showed that osteoblast activity, measured as bone formation and mineral apposition rates in vivo, and as alkaline phosphatase activity and mineralized nodule formation rate *in vitro,* is also lower in B6 vs. C3H (Sheng, M. H., et al., (2004). In vivo and in vitro evidence that the high osteoblastic activity in C3H/HeJ mice compared to C57BL/6J mice is intrinsic to bone cells. Bone 35, 711-719). The B6 strain is therefore a useful and well-characterized model of generalized osteopenia. Adult low bone mass of this type also identifies the human population at greatest risk of osteoporosis, making the B6 strain a suitable model in which to test therapeutic and/or preventive strategies.

Three-month-old male and female C57B1/J6 mice are used in this study (The Jackson Laboratory, Bar Harbor, ME). The effect of oral administration of yeast cell wall particles (YCWP) loaded with hOPG expression constructs (YCWP-hOPG) is determined by radiography, micro-CT and pQCT, and by immuno- and enzyme histochemistry. YCWP or yeast cell wall particle is a generic description of the particles, encompassing YGMP and YGP.

Animals are randomly assigned to control or experimental groups. One group of at least 10 animals is fed YCWP-hOPG constructs designed as described below, while another group is fed YCWP loaded with vector DNA. A suitable dose is about roughly 400 µg/day, modified as necessary to obtain the desired effect X-rays are taken every two weeks to monitor progress. Treatment is continued for 2 months, at which time point, animals are sacrificed for micro-CT analysis of the femur and histological analysis of the tibia. High-resolution, whole body X-rays are obtained (Faxitron Micro 50), femurs dissected free of extraneous tissue and fixed overnight in cold formaldehyde in PBS, after which they are switched to 70% ethanol for micro-CT analyses. Tibiae are split longitudinally, fixed in 4% paraformaldehyde, demineralized in EDTA and processed for paraffin embedment for subsequent immuno- and histochemical analysis. Some sections are immunostained for hOPG expression and the macrophage F4/80 marker, and analyzed by FACS to assess macrophage expression of hOPG. Other sections are stained for the osteoclast marker, TRAP, and TRAP-positive cells are counted in the proximal tibial metaphysis. TRAP-positive cells in two fixed areas of the proximal tibial metaphysis are counted by at least two observers in at least three sections from each animal and the results from the experimental groups will be tested for significant difference of the means by t-test. Total and volumetric bone mineral density of the femur are measured by peripheral quantitative computed tomography (pQCT) with a Stratec XCT 960M instrument. Thresholds for distinguishing non-bone from other tissues, and of cortical bone from lower density bone as well as calculation for total cortical thickness, are as previously described for this type of osteopenic mouse. Bone microarchitecture is assessed by micro-CT, also as described (Turner et al., 2001) using a desktop micro-CT instrument (iCT 20, Scanco Medical, Basserdorf, Switzerland). The resulting parameters are bone volume density, bone surface density, trabecular number, trabecular thickness, trabecular spacing, and trabecular number. Histological examination is performed on tibiae.

The Ovariectomized Mouse. The ovariectomized (OVX) mouse is another well-established and widely utilized model for studying low bone mass which mimics postmenopausal osteoporosis. Removal of ovaries from young (typically, 9-16 weeks old) adult female mice results in reproducible osteoporosis within several weeks due to accelerated osteoclastic bone resorption. Low bone density is most often measured as bone mineral density of either the femur or tibia along with pQCT determination of bone volume/total volume and trabecular thickness and number. Histomorphometric assessments may also be used to determine whether osteoclasts and osteoblasts per bone surface vary between experimental groups. In a recent example using this model, 3 weeks after OVX performed at 9 weeks, bone mineral density had decreased over 10%, bone volume/total, volume (BV/TV) had decreased roughly 40%, trabecular thickness had dropped by over 10%, and trabecular number was reduced by over 30% (Idris, A. L, et al., (2005) Regulation of bone mass, bone loss and osteoclast activity by cannabinoid receptors. Nat Med 11: 774-779). In another recent study, the OVX mouse model was used to assess the efficacy of adenoviral OPG gene therapy (Kostenuik, P.J., et al., (2004) Gene therapy with human recombinant osteoprotegerin reverses established osteopenia in ovariectomized mice. Bone 34:656- 664). The OVX mouse model has been similarly useful in many studies of low bone density and effects of therapeutic interventions. Thus, the OVX mouse is an acceptable model for testing the efficacy of orally ingested yeast cell wall particles loaded with hOPG-expression constructs to deliver hOPG to bone marrow.

Removal of ovaries from young (typically 9-16 weeks old) adult female mice is performed by standard procedures resulting in reproducible osteoporosis due to accelerated osteoclastic bone resorption within several weeks. Low bone density is measured in the femur as bone mineral density along with pQCT determination of bone volume/total volume and trabecular thickness and number. Typically three groups of mice are used in these studies: unoperated wild-type mice; sham operated wild-type mice; and, OVX operated mice. Sham operated mice have incisions, the ovaries are manipulated and then without removing the ovaries the incision is closed. Upon recovery from surgery, mice are fed either normal diets or gavaged daily with YCWP loaded only with vector DNA or treated with daily gavages of YCWP-hOPG compositions. Radiologic analyses are performed every two weeks, and after 6 weeks, animals are sacrificed and skeletal responses are assessed as described above for the C57B1/6 mice. Histomorphometric assessments can also be used to determine whether osteoclasts and osteoblasts per bone surface vary between experimental groups.

Recombinantly Generated Gaucher Mice. Skeletal complications are frequently observed in Gaucher disease and they are often difficult to treat. Long lived murine models of human Gaucher phenotypes are valuable for developing new therapeutic strategies (Xu YH, et al., (1996) Turnover and distribution of intravenously administered mannose-terminated human acid beta-glucosidase in murine and human tissues. Pediatr Res. 39(2):313-22; Willemsen R, et al. (1995) A biochemical and ultrastructural evaluation of the type 2 Gaucher mouse. Mol Chem Neuropathol. 24(2-3):179-92). The availability of these long lived L444P Gaucher mice having biochemical and phenotypic abnormalities, including osteopenia, similar to Gaucher patients having the same mutation provides a means to test the efficacy of the orally administered gene therapy in correcting the skeletal pathology observed in Gaucher disease (Hermann, G., et al., (1997) Gaucher disease: assessment of skeletal involvement and therapeutic responses to enzyme replacement. Skeletal Radiol 26:687-696). A transgenic mouse model of Gaucher disease was used in which amino acid substitutions were made in murine glucocerebrosidase that produced a significant reduction in endogenous GC expression to a level less than half that of the enzyme activity in normal littermates. Assay of glucocerebrosidase activity in mouse samples was performed using 4-methylumbellerferyl-glucopyranoside (4MUGP), a fluorescently labeled substrate. The point mutations, analogous to those found in the more mildly affected Gaucher disease patients, were introduced into a genomic clone of murine glucocerebrosidase by PCR mutagenesis. The modified clones were inserted into an appropriate vector and transfected into RW4 murine embryonic stem (ES) cells by electroporation. ES clones containing the correctly targeted mutation in one allele of the glucocerebrosidase gene were injected into blastocysts from C57BL/6 mice using standard techniques which were then transferred to foster mice. Male offspring from these injections were test-bred against C57BL/6 females, and the progeny were screened by PCR and Southern analyses for transmission of the mutant glucocerebrosidase allele.

The L444P, R463C and N370S mutations comprise three of the mutations most frequently found in Gaucher patients. The L444P mutation is found in higher frequency in patients having neurologic abnormalities. A replacement targeting vector using positive/negative selection was constructed containing a neomycin resistance (NeoR) cassette flanked by loxP sequences inserted into the intergenic regions between murine metaxin and glucocerebrosidase. The L444P mutation was introduced into a genomic clone of murine glucocerebrosidase by PCR mutagenesis. A construct was introduced into RW4 murine embryonic stem (ES) cells by electroporation and the ES cells were subjected to drug selection in culture with G418 as previously described. The correct gene.targeting event in G418 resistant individual clones was identified by Southern blot and PCR analysis. Cells from ES clones containing the correctly targeted L444P mutation in one allele of the glucocerebrosidase gene were injected into blastocysts from C57BL/6 mice and then transferred to foster mice. Male offspring from these injections having more than 30% coat color chimerism were test-bred against C57BL/6 females, and progeny were screened by PCR and Southern analyses for transmission of the mutant L444P glucocerebrosidase allele. Two lines of mice containing the L444P mutant allele were identified, and the DNA sequence confirmed by direct sequencing of PCR amplified DNA containing the mutation introduced into exon 9. Mice heterozygous for the L444P mutant glucocerebrosidase gene were mated and homozygous mutant progeny were identified by Southern blot and PCR analysis. In addition, heterozygous L444P mice were mated to mice carrying a transgene for CRE DNA recombinase, resulting in the excision of the NeoR marker, leaving only a 34 bp loxP sequence. The targeted L444P mutation was transmitted in a Mendelian fashion. Assay of glucocerebrosidase activity in mouse tail samples using 4-methylumbellerferyl-glucopyranoside (4MUGP), a fluorescently labeled substrate, demonstrated that in homozygous mutant mice the glucocerebrosidase activity was approximately 35% of the enzyme activity in normal littermates.

Osteoprotegerin Knockout Mice. Mice homozygous for the targeted disruption of OPG are valuable for studying the pathogenesis of osteoporosis, as well as an important resource for development of new therapies for low bone density. Typical of severe osteoporosis, homozygous mice older than eight weeks have significantly decreased trabecular bone in femurs and reduced bone mineral density, dry weight, mineral content, stiffness and strength compared to that of wild-type litter mates. The severe bone abnormalities observed in OPG -/- homozygous mice are accompanied by markedly increased numbers of osteoclasts. In contrast to wild-type or heteozygote littermates, abundant osteoclasts are present throughout the trabecular and cortical bones in OPG -/- mice. Both TRAP and osteopontin staining, as well as calcein in the mineralization fronts of eiphyses have been reported to be increased in bone from OPG -/- compared to wild-type parental strain C57BL/6J mice. Thirteen-week-old OPG-/- mice have a decrease in tail, distal femur and tibia bone radiodensity. Micro CT of the OPG-/- mice shows absence of trabecular bones, destruction of growth plates and abnormal femur cortical bone. The bone abnormalities seen in the OPG -/- mice are typical of severe osteoporosis.

A colony of OPG-/- mice was established using a male mouse carrying an OPG knockout allele that was generously provided by Dr. Michael J. McKenna and Arthur G. Kristiansen in the Department of Otology and Laryngology, Harvard Medical School, Boston, MA. The OPG functional gene knockout line was generated by targeted disruption of exon 2 in the murine OPG gene and backcrossing founder mice to the parental B6 strain. Mizuno, A., et al., Severe osteoporosis in mice lacking osteoclastogenesis inhibitory factor/osteoprotegerin, Biochem Biophys Res Commun. 1998 Jun 29;247(3):610-5. The severe abnormalities of bone remodeling and osteoporosis observed in these homozygous OPG-/- mice provide an excellent model for determining cellular and molecular mechanisms of altered bone remodeling and skeletal fragility, as well as an invaluable resource for the development of treatments for osteoporosis.

Osteoclast differentiation *in vitro.* Preliminary studies with the J774 cell line is extended to primary mouse bone marrow cell cultures to assess hOPG expression in bone marrow monocytes, macrophages and differentiating osteoclasts. YCWP are efficiently phagocytosed and retained by osteoclast precursors without evident ill-effect on cell survival or differentiation. Osteoclast differentiation is carried out as described below. Fresh bone marrow is obtained from normal mice at 2-4 weeks of age. Mononuclear cells are separated by gradient centrifugation on Histopaque 1077 (Sigma). The cells are then washed, resuspended in α-MEM supplemented with10% FBS (Invitrogen Life Technologies, Grand Island, NY) and 1% antibiotic/antimycotic (Sigma), and incubated at a density of 3x10⁵ cells/ml for 24 h in a 75 cm² flask (Coming) for 24 hours, after which the non-adherent cells are harvested by gentle agitation. This cell fraction is plated at a density of roughly 5 x 10⁵ cells per well in 12-well plates (or proportionately for other culture vessels) in osteoclast differentiation medium: α-MEM containing 10% FBS, antimycotic/antibiotic solution (Sigma), 75ng/mL CSF-1 (Chiron) and 30 ng/mL recombinant mouse RANK ligand (R&D Systems). The cultures are incubated at 37°C in a humidified atmosphere of 95% air and 5% CO2 for 6 days with the medium changed every other day, at which time many large, multinucleated cells can be observed.

Bone marrow monocytes, macrophages and differentiated osteoclasts are immunostained for hOPG expression and cell-type markers, and analyzed for hOPG expression in each cell-type. The hOPG secreted into the medium is determined using a commercially available ELISA kit (Immunodiagnostic Systems; BioVendor). Osteoclasts are counted as tartrate-resistant acid phosphatase (TRAP)-positive, multinucleated cells as described. Cells are fed YCWP at the time of plating, before osteoclast differentiation has occurred. At various times, wells are collected for TRAP-staining (p-nitrophenolphosphate method; and for RNA and protein extraction. hOPG mRNA is determined by real-time RT-PCR using a Light Cycler system (Roche) using SYBR green incorporation normalized to GAPDH.

Radiologic Analyses. Total and volumetric bone mineral densityof the femur are measured by peripheral quantitative computed tomography (pQCT) with a Stratec PCT 960M instrument. Thresholds for distinguishing non-bone from other tissues, and of cortical bone from lower density bone as well as calculation for total cortical thickness, are as previously described for this type of osteopenic mouse. Bone microarchitecture will be assessed by micro-CT, also as described (Turner et al., 2001) using a desktop micro-CT instrument (iCT 20, Scanco Medical, Basserdorf, Switzerland). The resulting parameters are bone volume density bone surface density, trabecular number, trabecular thickness, trabecular spacing, and trabecular number. Histological examination is performed on tibiae.

Analysis of Systemic Tissues. Measurements of human OPG in tissues provide data on the time course and levels to which the orally administered macrophage/ osteoclast targeted gene therapy results in expression of OPG in mouse tissues. ELISA, Western blot and RT qPCR measurements provide information on enzyme restoration at both the transcript and protein levels. Immunohistochemical and electron microscopy analyses provide data on the extent of osteoclast population in tissues.

Analysis of Bone Tissue. Measurements of human OPG in different locations of bone in mice provide data on the time course and levels to which the macrophage/osteoclast targeted gene therapy results in OPG expression. ELISA, Western blot and RT qPCR measurements will provide information on OPG at both the transcript and protein levels. Immunohistochemical and electron microscopy analyses using samples from different bone locations provide data on the numbers and location of macrophages expressing human OPG,

Evaluation of the Phenotype. The clinical status of wild-type and low bone density mice is followed in order to detect any changes resulting from OPG gene therapy. Mice are observed for neurologic, gait and other abnormalities. As appropriate, mice undergo behavior and motor testing. Physiologic tests on these mice includes routine blood chemistry and hematology.

Tissue Harvesting. At the tissue sampling points of the experiments, animals are euthanized using approved protocols and tissue samples are collected from all organs (e.g. bone, bone marrow, spleen, thymus, liver, lung, heart, kidney, brain, etc) and either frozen or fixed for analyses. Tissues are analyzed for expression of human OPG. The assays include ELISA, real time qPCR, Southern blot, Northern blot, and immunohistochemistry.

Tissue Extraction for Assays. Tissues are homogenized (20% wt/vol) in phosphate buffered saline (pH 7.5) containing 0.1% Triton X-100. The tissue homogenates are centrifuged at 40C at 48,000 X g for 20 minutes, and the supernatants are stored at -20 degrees Celsius. Protein content is determined by the method of Bradford.

Osteoprotegerin ELISA Assay. The majority of OPG produced in vitro in tissue culture cells is secreted into the medium and therefore both the cells and the culture medium are assayed for OPG. The human osteoprotegerin ELISA is a biotin labeled antibody based sandwich enzyme immunoassay providing a quantitative measurement of human osteoprotegerin in serum, plasma, synovial fluid or tissue culture medium (BioVendor LLC, Candler, NC). In this human osteoprotegerin ELISA, the standard or sample is incubated with a mouse monoclonal anti-human osteoprotegerin antibody coated in microtiter wells. After one-hour incubation and a washing, biotin-labeled polyclonal anti-human osteoprotegerin antibody is added and incubated with captured OPG. After a thorough wash, streptavidin horseradish peroxidase conjugate is added. After an half hour incubation and a final washing step, the bound conjugate is reacted with the substrate, H₂O₂-tetramethylbenzidine. The reaction is stopped by addition of acidic solution and the absorbance of the resulting yellow product is measured at 450 nm. The absorbance is proportional to the concentration of osteoprotegerin. The concentrations of unknown samples are determined using a standard curve generated by plotting absorbance values versus osteoprotegerin standard concentrations. The limit of detection (defined as the concentration of OPG giving absorbance higher than the mean absorbance of the blank plus three standard deviations of the absorbance of the blank: is better than 0.4 pmol/l of sample. There is only an approximately 1% cross-reactivity with recombinant mouse OPG, less than 0.06% with recombinant human CD40, rec. human sTNF RI or sTNF RII. A recombinant chimeric protein consisting of human osteoprotegerin and Fc-domain of human IgG (OPG/Fc) is used as standard. Mature OPG/Fc is a disulfide linked homodimeric protein. Each monomer contains 380 residues from mature OPG and 243 residues from the Fc protein and linker. As a result of glycosylation, the OPG/Fc migrates as a 77 kDa protein (previously it was referred to as 100 kDa) in SDS-PAGE under reducing conditions.

Immunoprecipitation. To determine the increase in human OPG in the proposed in vivo and in vivo gene transfer experiments, human OPG cell and tissue extracts are purified using a Protein G immunoprecipitation kit according to the manufacturer's procotol (Sigma). A polyclonal or monoclonal antibody to human OPG is used for this immunoprecipitation procedure, followed by Western blot analysis.

Blood Analyses. Mouse blood is obtained by tail vein or retro-orbital bleeding of mice for routine chemistry, hematology, as well as for other assays, including OPG ELISA.

Bone Histology. For routine histological assessments, tibiae are fixed overnight in cold 4% paraformaldehyde and demineralized in EDTA, after which they are embedded in paraffin. Sections are stained with either H&E or toluidine blue, or used in the immunohistochemical experiments described below. For mineralization assessments, non-demineralized paraffin blocks are cut in a cryotome and morphometric measurement of mineralized bone in the tibial metaphysis are made on Von Kossa stained sections using digital micrographs and image analysis software (Zeiss Axiovision and Osteomeasure). Some sections are stained with Masson's trichrome to visualize osteoid vs mineralized bone matrix.

Histology. Non-skeletal tissue samples for histologic analyses are fixed in 10% formalin overnight, rinsed in PBS, dehydrated through increasing graded strengths of ethanol, cleared and embedded in paraffin, and cut into 5 micron sections. Serial sections are stained with hematoxylin and eosin.

Immunohistochemistry. Wild-type and low bone density mice are euthanized and the harvested tissues are fixed in 4% paraformaldehyde in phosphate-buffered saline, pH 7.4, overnight, and embedded in paraffin. Tissue sections for immunohistochemistry are cut on a cryostat (5-10 microns), plated on glass slides and deparaffinized and rehydrated. Sections are treated with 5% H₂O₂ in PBS for 5 minutes to inhibit endogenous peroxidase. Following incubation in 1% bovine serum albumin/PBS for 60 minutes to prevent nonspecific binding, sections are processed using polyclonal or mouse monoclonal antibodies specific for human OPG, biotinylated goat-anti-rabbit or goat-anti-mouse secondary serum, and ABC complex (Vectastain Elite kit, Vector) and visualized with DAB chromagen according to the manufacturer's protocol. Images are captured with a Zeiss microscope equipped with a CCD camera and Scanalytics software. Immunostaining without primary antibody, or using preimmune antisera, is used as negative control.

Electron Microscopy. Election microscopic analyses permit further description of the cellular source of OPG expresion, as well as characterization of alterations in osteoclast structure that result from OPG gene therapy. Tissue samples for routine electron microscopy are fixed in glutaraldehyde. For immunoelectron microscopy samples are fixed as previously described and immunostained by incubation with anti-human OPG antibody.

*In-situ* Hybridization. *In-situ* hybridization studies are performed on treated and untreated mice at 1, 3, 6 and 12 months as part of the determination of extent and duration of human OPG expression in tissues. Mice are anesthetized and then perfused with physiological saline followed by 4% paraformaldehyde in PBS. Processing of bone tissue is as described in by Marks, Jr., S.C., et al., (1999) Facial development and type III collagen RNA expression: concurrent repression in the osteopetrotic (toothless, tl) rat and rescue after treatment with colony-stimulating factor-1. Dev. Dyn. 215: 117-125. Other tissues are excised and immersed in 4% paraformaldehyde in PBS for 1 hr, paraffin embedded and 5 µm sections mounted on slides. The sections are deparaffinized, rehydrated, dehydrated and dried. Digoxigenin-labeled sense and antisense riboprobes for in-situ analyses are generated from 600-700bp subcloned fragments of mouse or human OPG cDNAs using an AmpliScribeTM T7 high yield transcription digoxigenin RNA labeling kit (Epicentre, Inc.) as as previously described (Odgren, P.A., et al., (2003) Production of high-activity digoxigenin-labeled riboprobes for in-situ hybridization using the AmpliScribe T7 high yield transcription kit. Epicentre Forum 10: 6-7). Tissue sections embedded in paraffin are deparaffinized, hybridized with DIG-labeled probe diluted 1:200 in hybridization mix (50% formamide, 5xSSC, 10% dextran sulfate, 1x Denhart's solution, 1 µl/ml RNAse inhibitor and 500 µg/ml tRNA), and detected with an anti-digoxigenin antibody coupled to alkaline phosphatase and the colorimetric substrate NBT/BCIP (nitrobluetetrazole/5-bromo-4-chloro-3-indolyl phosphate) according to protocol (Roche). Control hybridizations are carried out by treatment of sections with RNase A (100 ug/ml) for 30min at 37C before hybridization with antisense probes.

Western Blot Analysis. Western blot analysis can be performed to confirm that gene transfer results in expression of human OPG in cells and medium in vitro and in mouse tissues *in vivo.* Samples are extracted as described above. Prior to electrophoresis, the protein concentrations of samples are measured (BioRad protein assay). Samples (25 µg total protein) are run on 12% SDS-PAGE, transferred to nitrocellulose membranes by electroblotting, and then incubated at RT for 60 min in 0.1% bovine serum albumin in PBS. The membranes are incubated with appropriate dilustions of antisera to human OPG in 0.1% bovine serum albumin in PBS at 4C overnight. After three washes for 5 min with PBS containing 0.05% Tween 20, the blots are processed using the Western Breeze kit (Invitrogen) as per manufacturer's protocol. Chemiluminescence is detected using XAR-5 film (Kodak).

RNA Extraction Protocols. Blood. Extraction of RNA from are accomplished using a QAIAmp RNA blood mini kit. Samples are treated with RNase-free DNase. RNA is extracted from frozen tissues using the animal tissue protocol in the RNAeasy Mini or Micro kit. Tissues are harvested, stored at -80C and ground under liquid nitrogen. After the lysis buffer is added to the ground tissue, the lysate is homogenized using a QIAshredder column and the Qiagen protocol is carried out as recommended, including RNase-free DNase treatment. Frozen tissues are transitioned with RNAlater-ICE and similarly processed. Integrity of the 28S and 18S rRNA bands is used to determine the intactness of each total RNA sample.

Real-time quantitative PCR. Real-time qPCR expression determinations are performed using an ABI 7900HT instrument on total RNA isolated using the Qiagen RNAeasy kit according to manufacturer's instructions. A DNAse I treatment before cDNA synthesis from 200ng of total RNA is used to remove genomic DNA. Random hexamers are used to initiate the 1 st strand synthesis in 20 µl using Qiagen Sensiscript reverse transcriptase enzyme according to the manufacturer's protocol. Each TaqMan assay is carried out in triplicate to measure transcription levels. These measurements provide data on the time course and levels of human and mouse OPG transcription following the orally administered gene therapy.

Northern Analyses. Total RNA is isolated from wild-type and treated and untreated mouse tissues using RNAeasy (Qiagen)and performed as per manufacturer's protocol. Eight micrograms of total RNA are loaded per lane on an 0.8% agarose formaldehyde gel, and the electrophoretically separated RNA transferred to nylon membranes (Hybond N, Amersham). Hybridization is carried out at 68°C for 1hr in ExpressHyb solution (BD Clontech), washed and autoradiography performed as per manufacturer's protocol. A ³²P labeled probe derived from a PCR fragment unique to mouse or human OPG is used for hybridizations, and a ³²P labeled alpha-actin and/or GAPDH probe is used for sample to sample normalization.

Laser Capture Microdissection. Laser Capture Microdissection (LCM) is used to obtain additional data at the molecular level on the cellular location, extent and duration of expression of human OPG within specific cell types (such as macrophages vs osteoclasts. These studies are performed using a PixCell IIe LCM System (Arcturus Inc.) and based on extensive experience with LCM for capture, isolation, amplification and quantitation of RNA and/or DNA from specific tissue targets. The LCM technique is compatible with a wide variety of slide fixation techniques, including frozen, formalin-fixed paraffin-embedded and fluorescently labeled sections. LCM is used to identify and navigate to cell populations of interest to obtain samples for DNA and/or RNA analyses. In brief, the process to capture cells and recover biomolecules using the PixCell IIe LCM System involves locating the cells of interest, followed by placing a LCM Cap over the target area. Pulsing the laser through the cap causes the thermoplastic film to form a thin protrusion that bridges the gap between the cap and tissue and adheres to the target cell. Lifting of the cap removes the target cell(s) now attached to the cap, and the captured cells are subsequently eluted into a 0.5 ml DNAase/RNAase free eppendorf microcentrifuge tube for further processing.

DNA Extraction Protocol Using CapSure Macro LCM Captured Samples. The CapSure Macro LCM Cap with the LCM captured cells are placed onto a 0.5 ml microcentrifuge tube containing 50 ul of proteinase K extraction solution. The microcentrifuge tube with the inserted CapSure Cap is inverted and gently shaken to ensure that the 50 µl volume of proteinase K solution completely covers the inside surface of the cap assembly. After incubation at 65°C the cap-tube assembly is centrifuged for 1 minute at 1,000 x g. The CapSure LCM cap is removed and the microcentrifuge tube containing the extract is heated at 95°C for 10 minutes to inactivate the proteinase K, cooled to room temperature, and used for PCR analysis.

RNA Extraction Protocol. In brief, RNA is prepared from cells captured on the CapSure HS LCM Caps using the PicoPure RNA Isolation Kit protocol as follows. Ten microliters of extraction buffer are added to the buffer well of the CapSure-ExtracSure assembly. A 0.5 mL microcentrifuge tube is placed onto the CapSure-ExtracSure assembly and the whole assembly incubated for 30 minutes at 42°C. The cell extract is collected by centrifuging the microcentrifuge tube with the CapSure-ExtracSure assembly at 800 x g for two minutes. The extract is then either used immediately for RNA isolation (see below) or stored at -80°C.

An RNA purification column is preconditioned with buffer for 5 minutes at room temperature and then centrifuged in a collection tube at 16,000 x g for 1 minute. After addition of 10 microliters of 70% ethanol to the cell extract, the cell extract with ethanol is added to the RNA purification column, centrifuged for 2 minutes at 100 x g, and then at 16,000 x g for 30 seconds to remove flowthrough. The purification column with bound RNA is washed with buffer, treated with DNAse, and washed again prior to elution of RNA with 11-30 µl of elution buffer. The isolated RNA is either amplified immediately or stored at -80°C until use. The amplified RNA resulting from PCR using primers specific for the human OPG construct is analyzed using agarose gel electrophoresis or quantitative realtime PCR to determine levels of expression.

Statistical Analysis. Data analyses are performed using statistical analysis software packages, including SigmaPlot, SAS (SAS Institute Inc., Cary, NC), NIH Image and SPSS software (SPSS Inc., Chicago, IL), generally using general linear regression and Student's t-test for analyses.

Preparation of WGP Particles. Whole Glucan Particles (WGP, Lot W0282) were previously obtained from Alpha-Beta Technology. In general, whole glucan particles are prepared from yeast cells by the extraction and purification of the alkaliinsoluble glucan fraction from the yeast cell walls. The yeast cells are treated with an aqueous hydroxide solution without disrupting the yeast cell walls, which digests the protein and intracellular portion of the cell, leaving the glucan wall component devoid of significant protein contamination, and having substantially the unaltered cell wall structure of β(1-6) and β(1-3) linked glucans. Yeast cells (*S. cerevisae* strain R4) were grown to midlog phase in minimal media under fed batch fermentation conditions. Cells (~90 g dry cell weight/L) were harvested by batch centrifugation at 2000 rpm for 10 minutes. The cells were then washed once in distilled water and them resuspended in 1 liter of 1M NaOH and heated to 90 degrees Celsius The cell suspension was stirred vigorously four 1 hour at this temperature. The insoluble material, containing the cell walls, was recovered by centrifuging at 2000 rpm for 10 minutes. This material was then suspended in 1 litter, 1M NaOH and heated again to 90 degrees Celsius. The suspension was stirred vigorously for 1 hour at this temperature. The suspension was then allowed to cool to room temperature and the extraction was continued for a further 16 hours. The insoluble residue was recovered by centrifugation at 2000 rpm for 10 minutes. This material was finally extracted in 1 liter, water brought to pH 4.5 with HCl, at 75 degrees Celsius for 1 hour. The insoluble residue was recovered by centrifugation and washed three times with 200 milliliters water, four times with 200 milliliters isopropanol and twice with 200 milliliters acetone. The resulting slurry was placed in glass trays and dried at 55 degrees Celsius under reduced pressure to produce 7.7g of a fine white powder.

A more detailed description of whole glucan particles and a process of preparing them can be found in U.S. Pats. Nos. 4,810,646; 4,992,540; 5,028,703; 5,607,677 and 5,741,495. For example, U.S. Pat. No. 5,028,703 discloses that yeast WGP particles can be produced from yeast cells in fermentation culture. The cells were harvested by batch centrifugation at 8000 rpm for 20 minutes in a Sorval RC2-B centrifuge. The cells were then washed twice in distilled water in order to prepare them for the extraction of the whole glucan. The first step involved resuspending the cell mass in 1 liter 4% w/v NaOH and heating to 100 degrees Celsius. The cell suspension was stirred vigorously for 1 hour at this temperature. The insoluble material containing the cell walls was recovered by centrifuging at 2000 rpm for 15 minutes. This material was then suspended in 2 liters, 3% w/v NaOH and heated to 75 degrees Celsius. The suspension was stirred vigorously for 3 hours at this temperature. The suspension was then allowed to cool to room temperature and the extraction was continued for a further 16 hours. The insoluble residue was recovered by centrifugation at 2000 rpm for 15 minutes. This material was finally extracted in 2 liters, 3% w/v NaOH brought to pH 4.5 with HCl, at 75 degrees Celsius for 1 hour. The insoluble residue was recovered by centrifugation and washed three times with 200 milliliters water, once with 200 milliliters dehydrated ethanol and twice with 200 milliliters dehydrated ethyl ether. The resulting slurry was placed on petri plates and dried.

Preparation of YGP Particles. *S. cerevisiae* (100 g Fleishmans Bakers yeast) was suspended in 1 liter 1M NaOH and heated to 55 degrees Celsius. The cell suspension was mixed for 1 hour at this temperature. The insoluble material containing the cell walls was recovered by centrifuging at 2000 rpm for 10 minutes. This material was then suspended in 1 liter of water and brought to pH 4-5 with HCl, and incubated at 55 degrees Celsius for 1 hour. The insoluble residue was recovered by centrifugation and washed once with 1000 milliliters water, four times with 200 milliliters dehydrated isopropanol and twice with 200 milliliters acetone. The resulting slurry was placed in a glass tray and dried at room temperature to produce 12.4 g of a fine, slightly off-white, powder.

Preparation of YGMP Particles. *S. cerevisiae* (75 g SAF-Mannan) was suspended in 1 liter water and adjusted to pH 12-12.5 with 1M NaOH and heated to 55 degrees Celsius. The cell suspension was mixed for 1 hour at this temperature. The insoluble material containing the cell walls was recovered by centrifuging at 2000 rpm for 10 minutes. This material was then suspended in 1 liter of water and brought to pH 4-5 with HCl, and incubated at 55 degrees Celsius for 1 hour. The insoluble residue was recovered by centrifugation and washed once with 1000 milliliters water, four times with 200 milliliters dehydrated isopropanol and twice with 200 milliliters acetone. The resulting slurry was placed in a glass tray and dried at room temperature to produce 15.6 g of a fine slightly off-white powder.

Preparation of YCP Particles. Yeast cells (*Rhodotorula* sp.) derived from cultures obtained from the American Type Culture Collection (ATCC, Manassas, VA) were aerobically grown to stationary phase in YPD at 30 degrees Celsius. *Rhodotorula* sp. cultures available from ATCC include Nos. 886, 917, 9336, 18101, 20254, 20837 and 28983. Cells (1L) were harvested by batch centrifugation at 2000 rpm for 10 minutes. The cells were then washed once in distilled water and then resuspended in water brought to pH 4.5 with HCl, at 75 degrees Celsius for 1 hour. The insoluble material containing the cell walls was recovered by centrifuging at 2000 rpm for 10 minutes. This material was then suspended in 1 liter, 1M NaOH and heated to 90 degrees Celsius for 1 hour. The suspension was then allowed to cool to room temperature and the extraction was continued for a further 16 hours. The insoluble residue was recovered by centrifugation at 2000 rpm for 15 minutes and washed twice with 1000 milliliters water, four times with 200 milliliters isopropanol and twice with 200 milliliters acetone. The resulting slurry was placed in glass trays and dried at room temperature to produce 2.7 g of a fine light brown powders.

Fig. 2 is a schematic diagram 100 of a transverse section of a yeast cell wall, showing, from outside to inside, an outer fibrillar layer 110, an outer mannoprotein layer 120, a beta glucan layer 130, a beta glucan layer- chitin layer 140, an inner mannoprotein layer 150, the plasma membrane 160 and the cytoplasm 170.

Fig. 3A is a schematic diagram of the structure of a YGP beta glucan particle 420, showing beta 1,3-glucan fibrils, the bud scar, which includes chitin, and and chitin fibrils. Fig. 3B is a schematic diagram of the structure of a YGMP beta glucan-mannan particle particle 430, showing beta 1,3-glucan fibrils, the bud scar, which includes chitin, mannan fibrils and chitin fibrils.

Table 1 summarizes the results of analyses of the chemical composition of WGP particles, YGP particles, YGMP particles and YCP particles that were prepared as described above. Note that YGP particles and YGMP particles have lower beta-glucan content, generally between about 6 to about 90 weight percent, and higher protein content compared to the prior art WGP particles. YGMP particles have a substantially higher mannan content, generally more than about 30 weight percent, more preferably between about 30 to about 90 weight percent mannan, compared to the other particle types. YCP particles have a substantially higher chitin + chitosan content compared to the other particle types, generally more than 50 weight percent, more preferably between about 50 to about 75 weight percent.

| Table 1 Chemical Composition of Yeast Cell Wall Materials | | | | | |
|---|---|---|---|---|---|
| Analyte | Method | WGP *S. cerevisiae* | YGMP *S. cerevisiae* | YGP *S. cerevisiae* | YCP *Rhodotorula* |
| Macromolecular | | | | | |
| Composition* | | | | | |
| Protein | Kjeldal | <1 | 4.5 | 4.9 | - |
| Fat | Base hydrolysis, Soxhlet extraction | <1 | 1.6 | 1.4 | - |
| Ash | Combustion | 1.2 | 1.9 | 1.6 | - |
| Carbohydrate | | | | | |
| Composition** | | | | | |
| Beta-Glucan | Enzymatic Hydrolysis | 90.3 | 41.9 | 77 | 6.5 |
| Chitin + chitosan | Monosac | 2.1 | 2.3 | 2.4 | 68 |
| (as glucosamine, n-acetyl glucosamine) | Analysis-Dionex | | | | |
| Mannan | Monosac | <1 | 36.9 | 0.47 | 1.3 |
| (as mannose) | Analysis- Dionex | | | | |
| Other Glucans | Monosac | 6.2 | 10.9 | 11.2 | 0.2 |
| (as non beta 1,3-glucose and other unmeasured sugars) | Analysis-Dionex | | | | |
| *Results are reported % w/w of dry analyzed materials | | | | | |
| **Results are reported % w/w carbohydrate | | | | | |
| WGP - Whole Glucan Particle - Prior Art Technology, YGMP - Yeast Glucan-Mannan Particle; YGP - | | | | | |
| Yeast Glucan Particle; YCP -Yeast Chitin Particle | | | | | |

### Exemplary Payload Trapping Molecules

### Preparation of Chitosan Loaded YGP Particles

YGP particles were prepared with a cationic trapping polymer, chitosan. 1% w/v chitosan solutions were prepared in 0.1M acetic acid using either High Molecular Weight (HMW) chitosan (~ 70,000 Mw, Sigma Chemical St. Louis, Mo) or Low Molecular Weight (HMW) chitosan (~ 10,000 Mw, Sigma Chemical St. Louis, Mo). Both 1% w/v HMW and LMW chitosan solutions were prepared in 0.1M acetic acid. Four ml HMW or LMW chitosan solution was added to 2 g YGP in a 50 ml conical centrifuge tube and mixed until a smooth paste was formed. The mixture was incubated for 1 hour at room temperature to allow the liquid to be absorbed. NaOH (40 ml, 0.1M) was added to each tube, which was vortexed immediately to precipitate the chitosan inside the YGP. The YGP:chitosan suspension was passed through an 18 gauge needle to produce a fine suspension of YGP:chitosan particles. The YGP:chitosan particles were collected by centrifugation (2,000 rpm for 10 minutes) followed by washing the pellet with deionized water until the pH of the supernatant was 7-8. The YGP:chitosan particles were then washed four times with two pellet volumes of isopropanol and then washed twice with two pellet volumes of acetone. The YGP:chitosan particles were then dried at room temperature in a hood. The procedure yielded 1.2 g YGP:LMW chitosan particles and 1.4 g YGP:HMW chitosan particles.

### Preparation of CytoPure™ Loaded YGP Particles (reference)

YGP particles were prepared with a biodegradable cationic trapping polymer, CytoPure™, a proprietary, commercially available, water-soluble cationic polymer transfection reagent (Qbiogene, Inc., CA). Twenty µl CytoPure™ was diluted in 0.5 ml deionized water and added to 0.5 g YGP in a 50 ml conical centrifuge tube and mixed until a smooth paste was formed. The mixture was incubated for 15 minutes at 4 degrees Celsius to allow the liquid to be absorbed. Twenty-five ml ethanol was added to each tube, which was vortexed immediately to precipitate the CytoPure™ inside the YGP. The YGP:CytoPure™ suspension was sonicated to produce a fine suspension of YGP:CytoPure™ particles. The YGP:CytoPure™ particles were collected by centrifugation (2,000 rpm for 10 minutes) followed by washing the pellet four times with two pellet volumes of isopropanol and then washed twice with two pellet volumes of acetone. The YGP:CytoPure™ particles were then dried at room temperature in a hood. The procedure yielded 0.45 g YGP:CytoPure™ particles.

### Preparation of Polyethylenimine Loaded YGP Particles

YGP particles were prepared with polyethylenimine (PEI) as a cationic trapping polymer. A 0.5 ml aliquot of a 2% w/v PEI (~ 50,000 Mw, Sigma Chemical Co., St. Louis, MO) solution in water was added to 0.5 g YGP in a 50 ml conical centrifuge tube and mixed until a smooth paste was formed. The mixture was incubated for one hour at room temperature to allow the liquid to be absorbed. Twenty-five ml ethanol was added to each tube, which was vortexed immediately to precipitate the PEI inside the YGP. The YGP:PEI suspension was passed through an 18 gauge needle to produce a fine suspension of YGP:PEI particles. The YGP:PEI particles were collected by centrifugation (2,000 rpm for 10 minutes) followed by washing the pellet four times with two pellet volumes of isopropanol and then washed twice with two pellet volumes of acetone. The YGP:PEI particles were then dried at room temperature in a hood. The procedure yielded 0.48 g YGP:PEI particles.

### Preparation of Alginate Loaded YGP Particles (reference)

YGP particles were prepared with alginate (F200 or F200L, Multi-Kem Corp., Ridgefield, NJ) as an anionic trapping polymer. A 2 ml aliquot of a 1% w/v alginate solution in water was added to 1 g YGP in a 50 ml conical centrifuge tube and mixed to form a smooth paste. The mixture was incubated for one hour at room temperature to allow the liquid to be absorbed. The mixture was diluted with 40 ml of a 1% w/v calcium chloride aqueous solution. The YGP:alginate suspension was passed through an 18 gauge needle to produce a fine suspension of YGP:alginate particles. The YGP:alginate particles were collected by centrifugation (2,000 rpm for 10 minutes. The YGP:alginate particles were washed four times with two pellet volumes of isopropanol and then washed twice with two pellet volumes of acetone. The YGP:alginate particles were then dried at room temperature in a hood. The procedure yielded 0.95 g YGP:F200 alginate particles and 0.86 g YGP:F200L alginate particles.

### Preparation of Poly-L-lysine Loaded YGP and YGMP Particles

YGP and YGMP particles were prepared with Poly-L-lysine (PLL) as a trapping polymer. A 4 ml aliquot of a 1% w/v PLL (Sigma Chemical Co., St. Louis, MO) solution in water was added to 1 g YGP or YGMP in a 50 ml conical centrifuge tube. The mixture was incubated for 30 minutes at 55 degrees Celsius to allow the liquid to be absorbed. Ten ml ethanol was added to each tube, which was homogenized (Polytron homogenizer) to produce a fine suspension of YGP:PLL or YGMP:PLL particles. The YGP:PLL or YGMP:PLL particles were collected by centrifugation (2,000 rpm for 10 minutes. The YGP:PLL or YGMP:PLL were washed four times with two pellet volumes of isopropanol and then washed twice with two pellet volumes of acetone. The YGP:PLL or YGMP:PLL particles were then dried at room temperature in a hood. The procedure yielded 1.3 g YGP:PLL particles and 1.1 g YGMP:PLL particles. Microscopic evaluation showed no free PLL aggregates, only YGP:PLL or YGMP:PLL particles.

### Preparation of Xanthan Loaded YGP and YGMP Particles (reference)

YGP and YGMP particles were prepared with xanthan as an anionic trapping polymer. A 4 ml aliquot of a 1% w/v xanthan solution in water was heated to 55 degrees Celsius to reduce viscosity and added to 1 g YGP or YGMP in a 50 ml conical centrifuge tube. The mixture was incubated for 30 minutes at 55 degrees Celsius. Ten ml ethanol was added to each tube, which was homogenized (Polytron homogenizer) to produce a fine suspension of YGP:xanthan or YGMP:xanthan particles. The YGP:xanthan or YGMP:xanthan particles were collected by centrifugation (2,000 rpm for 10 minutes). The YGP:xanthan or YGMP:xanthan particles were washed four times with two pellet volumes of isopropanol and then washed twice with two pellet volumes of acetone. The YGP:xanthan or YGMP:xanthan particles were then dried at room temperature in a hood. The procedure yielded 1.2 g YGP:xanthan particles and 1.1 g YGMP:xanthan particles. Microscopic evaluation showed no free xanthan aggregates, only YGP:xanthan or YGMP:xanthan particles.

### Use of YGP:Agarose to Trap Molecules by Physical Entrapment (reference)

YGP:Agarose was prepared to evaluate physical entrapment as a means to trap a payload in YGP. A 2% w/v solution of agarose (Sigma Chemical Co., St. Louis, MO) was prepared in TE, and cooled to 50 degrees Celsius. A 1 mg/ml stock solution of salmon sperm DNA in TE was diluted to 0.5 mg/ml DNA in TE or in 1% agarose at 50 degrees Celsius. A 500 mg aliquot of YGP was mixed with 500 µl of DNA in TE or 500 µl of DNA in agarose at 50 degrees Celsius and the mixture was incubated 1 hour at 50 degrees Celsius. The mixture was then cooled for 1 hour in a refrigerator to solidify the agarose. After 1 hour, 10 mls ofTE was added and the mixture was incubated overnight in refrigerator. The mixture was then centrifuged, and DNA in the supernatant was measured by absorption at 260 nm. About >80% of the applied DNA was retained by YGP:Agarose compared to <1% retained by the YGP:TE control. These results indicate that agarose effectively traps DNA inside YGP by physical entrapment.

### Use of YGP:Polyacrylamide to Trap Molecules by Physical Entrapment (reference)

YGP:Polyacrylamide was prepared to evaluate physical entrapment as a means to trap a payload in YGP. A 1mg/ml stock solution of salmon sperm DNA in TE was diluted to 0.5 mg/ml DNA in TE or in 30% polyacrylamide/bis (Sigma Chemical Co., St. Louis, MO). TEMED (N,N,N',N'-Tetramethylethylenediamine) was added to each DNA mixture (1 µl TEMED to 5 mls of DNA solution), and a 2 ml aliquot of each solution was added to 1 g YGP. The result was mixed to form a uniform suspension and incubated 3 hours at room temperature. After the 3 hour incubation, 10 ml of TE was added and the mixture was incubated overnight in a refrigerator. The mixture was then centrifuged, and DNA in the supernatant was measured by absorption at 260 nm. About >95% of the applied DNA was retained by YGP:Polyacrylamide compared to <1% retained by the YGP:TE control. These results indicate that polyacrylamide is an effective trapping polymer to use to trap DNA inside YGP by physical entrapment.

### Loading of Protein into YGP (reference)

The utility of the delivery system of the present invention for the retention, transport and delivery of therapeutic peptides or proteins, vaccine antigens or other peptides or proteins was evaluated using the mixed proteins of fetal calf serum. Yeast cell wall particles used were YGP, YGP-PEI and YGP-chitosan prepared as described above. Stock solutions were 45 ng/µl fetal calf serum (FCS) (Fetal Bovine Serum, JRH Biosciences, Lenexa, KS), 0.2% PEI (Sigma Chemical Co., St. Louis, MO) in TE, 0.05 M phosphate buffer, pH 7.2 (P buffer) and 0.05 M phosphate buffer, pH 7.2, 1M NaCl (P + salt buffer).

Four µl of FCS were added to 1 mg of YGP, YGP-P or YGP-CN in microcentrifuge tubes as indicated in Table 8 and the resulting mixture was incubated 60 minutes at room temperature to allow the liquid to be absorbed by the particles. After the incubation, 200 µl phosphate buffer or 200 µl PEI was as indicated in Table 8 and the resulting mixture was incubated 60 minutes at room temperature. After the incubation, 0.5 ml phosphate buffer was added, and after a further 5 minute incubation, the tubes were sonicated to produce single particles. The particles were pelleted by centrifuging at 10, 000 rpm for 10 minutes and the supernatants were removed to fresh tubes. 0.5 ml 0.05M sodium phosphate buffer, pH 7.2 + 1M NaCl was added to the pellets, and after a further 5 minute incubation, the tubes were centrifuged at 10, 000 rpm for 10 minutes and the high salt elution supernatants were removed to fresh tubes. The protein content of the supernatants was measured by absorbance at 280 nm.

The protein loading results are shown in Table 2, below. YGP particles without a trapping molecule trapped only 5% of the presented protein. YGP particles that were loaded first with FCS protein and then exposed to PEI retained 47% of the protein load. YGP particles that were preloaded with a trapping polymer such as PEI or chitosan before exposure to the protein load such retained 68% and 60%, respectively, of the protein load.

| **Table 2** | | | | | | | |
|---|---|---|---|---|---|---|---|
| **Tube** | **YGP** | **Payload** | **Trapping Polymer** | **Unbound Protein (ng)** | **% Unbound Protein** | **Unbound Protein (ng)** | **% Unbound Protein** |
| 1 | - | FCS | P buffer | 180 | 100 | - | - |
| 2 | YGP | FCS | P buffer | 180 | 95 | 10 | 5 |
| 3 | YGP | FCS | 2% PEI | 120 | 63 | 70 | 47 |
| 4 | YGP-PEI | FCS | P buffer | 60 | 32 | 130 | 68 |
| 5 | YGP-CN | FCS | P buffer | 80 | 40 | 120 | 60 |

The results demonstrate that serum proteins are not effectively loaded and trapped into YGP without trapping polymers. Using YGP that were preloaded with trapping polymers before exposure to the payload proteins resulted in increased protein trapping. Alternatively, proteins can be trapped inside YGP by first loading the protein, and then adding a soluble trapping polymer to sequester the protein within the particle.

### Fluorescently Labeled Plasmid DNA Loading and Trapping

GP containing fluorescent plasmid DNA compositions were prepared to optimize DNA trapping and to evaluate DNA delivery and release following uptake into J774 cells, a murine macrophage derived cell line. Fluorescent pUC 19 plasmid DNA was prepared by mixing 1 ml of a 1 mg/ml solution of pUC 19 DNA in 0.1M carbonate buffer pH 9.2 with 100 µl of a 1 mg/ml suspension of DTAF in 10 mM carbonate buffer pH 9.2. After overnight incubation at 37°C, 200 µl 1M Tris-HCl, pH 8.3 was added and incubated for 15 minutes at room temperature. Then 100 µl 1M NaCl and 3 ml ethanol were added to ethanol precipitate the DNA. After storage at -20 degrees Celsius overnight, the ethanol precipitate was collected by centrifugation at 10,000 rpm for 15 minutes. The ethanol precipitate was washed with 70% ethanol until the supernatant was clear, and resuspended in 1 ml TE.

Fluorescent DNA (1 µg/µl) was absorbed into dry YGP for 30 minutes at room temperature. After the incubation, 0.45 ml 95% ethanol was added to one tube, 0.2 ml 2% polyethylenimine (PEI), was added to two tubes and 0.2 ml 2% hexadecyltrimethyl-ammonium bromide (CTAB) was added to another tube. After 30 minutes incubation at room temperature, 0.2 ml 2% CTAB was added to one of the PEI tubes and incubation continued for 30 minutes. Ethanol (1ml, 95%) was added and the YGP-DNA compositions were stored overnight at -20 degrees Celsius. The YGP-DNA suspensions were washed with 70% ethanol and resuspended in 0.5 ml PBS. J774 murine macrophages were plated in six well plates at a density of 2.5 x105 cells per well and incubated overnight The particles were added to the culture medium at a 10 particle per cell ratio and the plates were swirled to distribute particles. The cells were incubated for 4 hours. At end of the incubation period, the culture medium was removed; the cells were washed with PBS and fixed in 0.4% formalin in PBS. Microscopic examination revealed that fluorescent particles had been taken up by the cells.

In other studies, YGP containing pIRES plasmid was prepared for transfection and expression of encoded EGFP in J774 cells. Cationic trapping agents used included cationic polymers such as polyethylenimine (PEI), CytoPitre™, a proprietary, commercially available, water-soluble cationic polymer transfection reagent (Qbiogene, Inc., CA), chitosan and a cationic detergent hexadecyltrimethylammoniumbromide (CTAB). A preferred PEI is JetPEI, a commercially available linear polyethylenimine cationic polymer transfection reagent (Qbiogene, Inc., CA).

pIRES-EGFP (Clonetech, CA) contains the internal ribosome entry site (IRES) of the encephalomyocarditis virus (ECMV) between the MCS and the EGFP (enhanced green fluorescent protein) coding region. This permits both the gene of interest (cloned into the MCS) and the EGFP gene to be translated from a single bicistronic mRNA. pIRES-EGFP is designed for the efficient selection (by flow cytometry or other methods) of transiently transfected mammalian cells expressing EGFP and another protein of interest. To optimize the selection of cells expressing high levels of the protein of interest, pIRES-EGFP utilizes a partially disabled IRES sequence. This attenuated IRES leads to a reduced rate of translation initiation at the EGFP start codon relative to that of the cloned gene. This enables the selection of those cells in which the mRNA, and hence the target protein, is produced at high levels to compensate for a suboptimal rate of translation of EGFP. This vector can also be used to express EGFP alone or to obtain stably transfected cell lines without time-consuming drug and clonal selection. EGFP is a red-shifted variant of wild-type GFP that has been optimized for brighter fluorescence and higher expression in mammalian cells. (Excitation maximum = 488 nm; emission maximum = 509 nm) EGFP encodes the GFPmut1 variant, which contains the amino acid substitutions Phe-64 to Leu and Ser-65 to Thr. These mutations increase the brightness and solubility of GFP, primarily due to improved protein folding properties and efficiency of chromophore formation. EGFP also contains an open reading frame composed almost entirely of preferred human codons. This leads to more efficient translation and, hence, higher expression levels in eukaryotic cells, relative to wild type GFP.

Solutions prepared were: pIRES EGFP plasmid DNA, 0.72 µg/µl in water, 0.2% w/v PEI (Sigma) in TE, 2 µl CytoPure (Qbiogene) + 48 µl 0.15M NaCl, 2 µl JetPEI (Qbiogene) + 48 µl TE, 0.2% Spermidine in TE, 2% (aq) CTAB and phosphate buffered saline (PBS).

Fluorescent pIRES plasmid DNA was prepared by mixing 1 ml of a 1 mg/ml solution of pIRES DNA in 0.1M carbonate buffer pH 9.2 with 100 µl of a 1 mg/ml suspension of DTAF in 10 mM carbonate buffer pH 9.2. After overnight incubation at 37 degrees Celsius, 200 µl 1M Tris-HCl pH 8.3 was added and incubated for 15 minutes at room temperature. Then 100 µl 1M NaCl and 3 ml ethanol were added to ethanol precipitate the DNA. After storage at -20 degrees Celsius overnight, the ethanol precipitate was collected by centrifugation at 10,000 rpm 15 minutes. The ethanol precipitate was washed with 70% ethanol until supernatant was clear and resuspended in 1 ml TE.

The YGP suspensions were incubated for 30 minutes at room temperature. After the incubation, 0.45 ml 95% ethanol was added to one set (YGP, YGP-P; YGP-Chitosan) of three tubes, 0.2 ml 2% PEI was added to two sets of three tubes and 0.2 ml 2% CTAB was added to another set of three tubes. After 30 minutes incubation at room temperature, 0.2 ml 2% CTAB was added to one set of the PEI tubes and incubation proceeded for a further 30 minutes. Ethanol (1ml, 95%) was added and the YGPs were stored overnight at -20 degrees Celsius. The YGP suspensions were washed with 70% ethanol and resuspended in 0.5 ml PBS.

J774 murine macrophages were plated in six well plates at a density of 2.5 x10⁵ cells per well and incubated overnight. The particles were added to the culture medium at a 10 particle per cell ratio and the plates were swirled to distribute particles. The cells were incubated for 4 hours. At end of the incubation period, the culture medium was removed, the cells were washed with PBS and fixed in 0.4% formalin in PBS.

Fluorescent DNA-containing particles and J774 cells incubated with fluorescent DNA-containing particles were evaluated by fluorescence microscopy, and results are summarized in Table 3.

| **Table 3** | | | |
|---|---|---|---|
| **Particle Type** | **Treatment** | **Color of Pellet** | **Microscopic Examination of Particles** |
| YGP | ethanol | White | No fluorescence |
| YGP-CN | ethanol | Yellow | Intracellular fluorescent particles |
| YGP-P | ethanol | Yellow | Intracellular fluorescent particles, |
| YGP | 2% PEI | Yellow | Intracellular fluorescent particles |
| YGP-CN | 2% PEI | Yellow | Intracellular fluorescent particles |
| YGP-P | 2% PEI | Yellow | Intracellular fluorescent particles |
| YGP | 2% CTAB | Yellow | Intracellular fluorescent particles |
| YGP-CN | 2% CTAB | Yellow | Intracellular fluorescent particles |
| YGP-P | 2% CTAB | Yellow | Intracellular fluorescent particles |
| YGP | 2% PEI/2% CTAB | Yellow | Strongly fluorescent Intracellular particles |
| YGP-CN | 2% PEI/2% CTAB | Yellow | Intracellular fluorescent particles |
| YGP-P | 2% PEI/2% CTAB | Yellow | Intracellular fluorescent particles |

### Example 1

EGFP Expression By J774 Murine Macrophages Incubated With YGP:pIRES

The pIRES plasmid DNA was not fluorescently labeled in this Example, rather the functional expression of the green fluorescent protein (GFP) encoded by pIRES was used as a demonstration of uptake of loaded yeast cell wall particles, intracellular release of the pIRES DNA and expression of the GFP as evidenced by the production of fluorescence.

The YGP: pIRES compositions were prepared as follows. DNA was prepared from dilutions in deionized water of 1 mg/ml stock. The indicated amount of DNA solution was added to YGP and incubated for at least 30 minutes to allow for liquid absorption. The indicated amount of 0.2% PEI in TE or 0.2% chitosan in acetate buffer was added and the mixture was allowed to incubate for 5 minutes before sonication to produce single particles. After a further incubation of at least 30 minutes, the indicated amount of 2% CTAB was added. After an additional 5 minute incubation, the tubes were vortex mixed and incubated again for at least 30 minutes. The indicated amount of 95% ethanol was added. Each tube was then mixed and stored at -20 Celsius overnight. The YGP:pIRES formulated particles were then centrifuged, washed twice in 70% ethanol, collected by centrifugation at 10,000 rpm for 5 minutes, resuspended in 0.5 ml sterile PBS and sonicated to produce single particles. The number of particles per ml was counted and each composition was and stored at -20 degrees Celsius.

J774 murine macrophages were plated in 6 well plates at a density of 2.5 x10⁵ cells per well and incubated overnight at 37 degrees Celsius. The transfections were performed as summarized in Table 4, below. The particles were added to the culture medium at a 10 particle per cell ratio and the plates were swirled to distribute particles. The cells were fed daily and incubated for 2 days. At end of the incubation period, the culture medium was removed the cells were washed with PBS and fixed in 0.4% formalin in PBS. Cells were examined using fluorescence microscopy (Fig. 5). The results are summarized in Table 4. Eighty nine percent of J774 cells took up YGP-F particles. EGFP expression was evident in >80% of J774 cells as punctate fluorescence in vacuoles.

Fig. 6A and Fig. 6B are images of color fluorescence photomicrographs of bone marrow macrophages showing uptake of YGP-FITC particles 520 (Fig. 6A) and in Fig. 6B, uptake of YGP-FITC particles 530 and staining specific for the macrophage marker F4/80 540.

| **Table 4** | | | | |
|---|---|---|---|---|
| **Well** | **Description** | **YGP/Cell** | **volume** | **Appearance** |
| 1A | No Treatment Control | 0 | - | No detectible GFP fluorescent particles |
| 1B | YGPF Particle Uptake Control | 10 | 10 µl 1/10 | Phagocytosis of fluorescent YGFP Particles |
| 1C | YGP empty PEI/CTAB Control | 10 | 11 µl 1/10 | No detectible GFP fluorescent particles |
| 1D | YGP empty Chitosan/CTAB Control | 10 | 5 µl 1/10 | No detectible GFP fluorescent particles |
| 1E | YGP piRES PEI/CTAB | 10 | 10 µl 1/10 | Fluorescent GFP expression in cells |
| 1F | YGP pIRES Chitosan/CTAB | 10 | 6.5 µl 1/10 | Fluorescent GFP expression in cells |

### Example 2

EGFP Expression By Murine RAW Cells Incubated With YCWP Co-delivery *in-vitro* of YCWP-Texas Red and pgWIZ-GFP was studied using murine RAW cells: Murine RAW 264.7 cells (ATCC, Manasas, VA, No. TIB-71™) were plated in 6 well plates as described above for J774 macrophages. YCWP-tRNA/PEI/CTAB particles (1X107) containing positively charged yeast tRNA/PEI/CTAB polyplexes were loaded with 0.5 mg pgWizGFP DNA (Gene Therapy Systems, San Diego, CA) by absorbing the anionic plasmid DNA onto the surface of the cationic tRNA/PEI/CTAB nanopolyplexes within the YCWP. Then, the YCWP-tRNA/PEI/CTAB/gWizGFP compositions were coated with PEI (5mg). This YCWP-DNA composition at a particle:cell ratio of 5 was mixed together with empty YCWP-TR (YCWP chemically labeled with Texas Red, Molecular Probes) at a particle:cell ratio of 5, and then incubated with the murine Raw cells to demonstrate YCWP uptake (red particles) and GFP expression (green diffuse fluorescence) within the same cell. As can be seen in the fluorescent photomicrograph in Fig. 7A and Fig. 7B, cells taking up YCWP-TR express GFP.

### Example 3

### In Vivo Oral Bioavailability of YGP and YGMP Particles in Mice

The effect of cell surface carbohydrate composition on oral bioavailability of yeast glucan particles was studied using fluorescently labeled yeast cell wall particles. Fluorescently labeled yeast glucan particles (YGP-F) and fluorescently labeled yeast glucan-mannan particles (YGMP-F) were prepared by reacting YGP and YGMP with dichlorotriazinyl aminofluorescein (DTAF), 20 mg/ml in DMSO, freshly prepared, in 0.1M borate buffer, pH 8 for 2 days at 37 degrees Celsius. Excess DTAF was quenched with 1 M Tris buffer, pH 8.3 and washed free of unreacted products by repeated washing with sterile PBS.

Aiquots (0.1ml) of YGP-F (1 mg/ml) and YGMP-F (2.5 mg/ml) were administered to mice (C57B1/6 wild- type) by oral gavage and subcutaneous injection for 5 days. Feces pellets were collected on day 5 from each group. The mice were euthanized on day 7, and tissues (brain, liver, spleen, bone marrow and small intestine) were harvested. Brain, liver, bone marrow and small intestine were placed into 10% paraformaldehyde fixative. Spleens were recovered into separate tubes on wet ice containing 50 ul sterile PBS. They were macerated with scissors and pressed through 70 micron screens to produce single cell suspensions. Splenic cells were plated at ~10⁶ cells per 12-well plate and incubated for 24 hours at 37 degrees Celsius to allow for attachment. After washing away unbound cells, the wells were scored for adherent cells (macrophages) with internalized fluorescent particles by fluorescence microscopy. The results demonstrate that both YGP-F and YGMP-F are orally bioavailable and systemically distributed by macrophages. Analysis of homogenized feces demonstrated the presence of ~20% of the number of administered fluorescent particles indicating that oral absorption was about 80% efficient.

Further studies showed that orally administered yeast cell wall particles containing pIRES DNA were incorporated in-vivo into macrophages that then expressed EGFP. Oral and subcutaneous administration to mice *in vivo* of compositions comprising yeast cell wall particles containing the pIRES expression vector were effective in producing transient expression of green fluorescent protein in murine splenic macrophages. The isolated splenic cells were harvested and cultured as described above, and adherent cells were formalin fixed, examined using fluorescence microscopy and photographed. Fluoresent photomicrographs of splenic macrophage cells demonstrated uptake of the YGMP:pIRES particles and expression of green fluorescent protein.

### Example 4

### Human Osteoprotegerin Expression By J774 Murine Macrophages Incubated With YGP:pIRES2DsRED2-OPG.

The payload molecule, pIRES2DsRED2-OPG plasmid DNA expressing human osteoprotegerin, was incorporated into yeast glucan particles (YGP) and yeast glucan-mannan particles (YGMP) in the form of cationic polymer-DNA nanocomplexes. Upon phagocytosis by macrophages, the particles are located in phagosomes where the cationic polymer swells the phagosome, releasing the DNA into the cytoplasm. The released DNA migrates to the nucleus and is processed by cellular machinery to produce active, normal osteoprotegerin.

Description of pIRES2DsRED2-OPG plasmid. The pIRES2DsRED2-OPG-OPG construct used in these preliminary experiments is comprised of human osteoprotegerin cDNA inserted between the *Bath*H1 and *Xho*1 sites of the pIRES2DsRED2 multiple cloning site (MCS). The pIRES2DsRED2 vector (Catalog No. 6990-1, Clontech Laboratories, Inc., Mountain View, CA) contains an IRES element and a CMV promoter that is responsible for expression of the human osteoprotegerin DNA insert sequence. Human OPG cDNA was cloned from a human kidney cDNA library and encodes a 401 amino acid polypeptide with features of a secreted glycoprotein, including a hydrophobic leader peptide and four potential sites of N-linked glycosylation.

YGP-DNA compositions deliver a plasmid DNA (pIRES2DsRED2-OPG) expressing human osteoprotegerin efficiently into a murine macrophage cell line J774. Methods as described above were used to load and trap pIRES2DsRED2-OPG expressing human osteoprotegerin into YGP. Adherent J774 cells in culture were incubated with YGP or YGP: pIRES2DsRED2-OPG at a 10 particle:cell ratio for 48 hours. The culture media was removed, the cells washed briefly with PBS, and then fixed (with 0.5-1% formalin solution). After removal of fixative, the cells were washed briefly with PBS and then incubated at RT for 1hr in 1.0% milk. After removal of the milk blocking solution the cells were then incubated at 4oC overnight with a mouse monoclonal antibody (Imgenex, IM103) specific for human osteoprotegerin (1/500 working dilution in PBS/1.0% milk). After overnight incubation, the antibody solution was removed from the cells, and the cells were washed 3 times for 5 min with PBS/0.05% Tween 20 with gentle rocking. The cells were then incubated with donkey anti-mouse Cy5 conjugated antisera (Molecular Probes, Cy5 donkey anti-mouse 2 mg/mL; 1/100-1/50 working dilution) for 1hr at RT. The cells were again washed five times for 3 min with PBS/0.05% Tween 20 with gentle rocking. After removal of the final wash solution, PBS was added to each well and the cell plates were stored at 4oC in the dark until fluorescent microscopic analysis.

Fig. 8A and Fig. 8B are images of color fluorescence photomicrographs of J774 cells sham transfected (Fig. 8A) or treated *in vitro* with YGP: pIRES2DsRED2-OPG (Fig. 8B). Human osteoprotegerin expression was detectable as immunoreactivity in >50% of J774 cells treated in vitro with YGP: pIRES2DsRED2-OPG compositions, such as indicated cell 610. The anti-human osteoprotegerin antibody selectively identified recombinant human osteoprotegerin and did not cross-react with endogenous mouse osteoprotegerin. These results demonstrate that YGP: pIRES2DsRED2-OPG compositions are effective in efficiently delivering the human osteoprotegerin encoding DNA, resulting in transient expression of human osteoprotegerin in murine J774 macrophage cells.

### Example 5

### Expression and Secretion of hOPG in Physiologically Significant Amounts

A composition of YGP (5x10⁷) loaded with 2 mg ofpIRES2DsRED2-hOPG DNA, coated with 10 mg polyethyleneimine (PEI, Aldrich) was used to transfect the 3T3-D1 murine fibroblast cell line in culture. As a positive control, cells were transfected with pIRES2DsRED2-hOPG DNA using a conventional transfection agent (JetPEI, Gene Therapy Systems, San Diego, CA). The pIRES2DsRED2-hOPG DNA (1 mg) in 50 ml 0.15M NaCl was mixed and immediately vortexed with either 2 or 4 mg JetPEI in 50 ml 0.15M NaCl. After incubation at RT for 20 minutes the transfection mixture was added to 3T3-D1 mouse fibroblast cells stably transfected with the murine dectin-1 gene were plated at 33% confluency in 24-well plates in DMEM with10% fetal calf serum (Invitrogen). Cells were transfected by adding 100 ml of YGP- DNA-PEI composition (20 ng DNA in 5x10⁵ particles) or 100 ml DNA-PEI polyplexes (1 mg DNA) dropwise to cells in 0.5 ml growth medium. Negative control wells were untreated. After incubation at 37° C in a CO₂ incubator for 3 hours, the growth medium was removed and replaced with 0.5 ml fresh D-MEM/10% fetal calf serum, and cells incubated as described above.

Aliquots of growth medium were removed and frozen at 24 and 48 hours and replaced with 0.5 ml fresh medium. An ELISA kit for hOPG (Cat. No. RD194003200; Immunodiagnostic Systems, BioVendor LLC, respresentative standard curve shown in Fig.9) was used to assay culture medium samples for expression and secretion of hOPG (Table 5).

| **Table 5: hOPG ELISA Analysis of Transfected Cell Medium at 48h** | | |
|---|---|---|
| Transfection | DNA (µg) | hOPG (pmole/l) |
| Untreated (Sham Transfection) | 0 | 0 |
| pIRES2DsRED2-hOPG JetPEI | 2 | 10.6-12.4 |
| YGP-pIRES2DsRED2-hOPG | 0.02 | 2.2 |

These data demonstrate that YCWP compositions can efficiently deliver DNA encoding hOPG that is secreted by cells as well as intracellularly expressed. Note that normalized for the amount of DNA presented, the delivery system of the present invention produced a twenty-fold greater amount of hOPG in the extracellular medium. These results demonstrate that transfection of 3T3-D1 cells with YCWP containing pIRES2DsRED2-hOPG DNA is efficient and results in the synthesis and secretion of hOPG into culture medium in physiologically significant amounts.

The results of these studies are summarized as follows. Murine macrophage J774 cells phagocytosed YGP-F particles efficiently (>90%). The anti-human osteoprotegerin antibody selectively identified recombinant human osteoprotegerin and did not cross-react with endogenous mouse osteoprotegerin. Human osteoprotegerin expression was detectable as immunoreactivity in >50% of J774 cells treated *in vitro* with YGP: pIRES2DsRED2-OPG compositions. YCWP compositions can efficiently deliver DNA encoding hOPG that is secreted by cells as well as intracellularly expressed. These results demonstrate that embodiments of the present invention are effective in efficiently delivering the human osteoprotegerin encoding DNA, resulting in transient expression of human osteoprotegerin in murine J774 macrophage cells and 3T3-D1 mouse fibroblast cells.

### Example 6

### Yeast Cell Wall Particles Administered To Mice Are Incorporated In-Vivo Into Macrophages And Abundantly Translocated To Bone

In a study to evaluate biodistribution of YGP to skeletal tissue, Texas Red (Molecular Probes) labeled YGP particles (YGP-TR) were administered to mice by intraperitoneal injection (IP; 1 mg/ml). The mice were euthanized on day 4, and tissues (brain, liver, spleen and bone) were harvested, fixed overnight in 5% formalin buffered with PBS and sections prepared for fluorescent microscope examination of tissue distribution of YGP-TR particles. The abundance of YGP-TR intracellular particles in the marrow space (some appearing contiguous to the endosteal surface) in a transverse section of mouse femur. Fig.10A - Fig.10C show images of tissue sections from a L444P Gaucher mouse that had received an IP injection of fluorescently labeled YGP particles four days previously, showing that fluorescently labeled particles 750 were distributed to bone. Fig. 10A shows a bone section viewed under transmitted light. Fig.10B shows the same field as in Fig. 10A viewed by fluorescence microscopy, showing several cells (arrows) that contain fluorescently labeled particles 750. Fig.10C is a higher magnification image that includes the field indicated by a rectangle in Fig. 10B. This study demonstrates that the YGP-DNA compositions administered to mice can be incorporated *in-vivo* into macrophages and abundantly translocated to skeletal tissue.

Fig.11 is a schematic diagram of a preferred embodiment of the method of delivering yeast beta glucan particles (YGP) 230 by macrophage migration 370 to various tissues after *in vivo* oral administration 180. A composition 182 containing yeast beta glucan particles (YGP) 230 is administered orally 180 to a subject 185. The yeast beta glucan particles (YGP) 230 are take up by M cells 355 in the lining of the small intestine and are translocated across the epithelium 350 and are phagocytosed by intestinal macrophages 360. The YGP-containing macrophages migrate 370 to organs and tissues including bone 450, lung 452, liver 454, brain 456 and spleen 458. About 72 hours after oral administration, splenic macrophages 364 that had phagocytosed YGP were observed in the spleen 458 (shown both schematically and in a reversed contrast grayscale image of a color fluorescence photomicrograph). About 90 hours after oral administration, bone marrow macrophages 362 that had phagocytosed YGP were observed in bone 450 (shown both schematically and in a reversed contrast grayscale image of a color fluorescence photomicrograph).

While the invention has been described in conjunction with the detailed description thereof, the foregoing description is intended to illustrate and not limit the scope of the invention, which is defined by the scope of the appended claims.

## Claims

1. A composition comprising:
a payload molecule that comprises a nucleic acid selected from the group consisting of an oligonucleotide, mRNA, a recombinant DNA construct, a linear DNA fragment, a blocked linear DNA fragment and, a mixture thereof, that encodes an anti-osteoclastogenic protein;
a payload trapping molecule selected from the group consisting of chitosan, polyethylenimine, poly-L-lysine, hexadecyltrimethylammoniumbromide and mixtures thereof; and
a carrier selected from a yeast glucan particle or a yeast glucan-mannan particle, wherein the carrier is an extracted yeast cell wall defining an internal space, the cell wall comprising 6 to 90 weight percent beta-glucan, wherein the payload trapping molecule is contained within the internal space defined by the carrier, and wherein the payload trapping molecule is present for facilitating retention of the payload molecule within the carrier.

2. The composition of claim 1, wherein the anti-osteoclastogenic protein is OPG or a functional equivalent thereof.

3. The composition of claim 1, wherein the recombinant DNA construct is an expression vector comprising a control element operatively linked to an open reading frame encoding an osteoprotegerin or a functional equivalent thereof.

4. The composition of claim 1, wherein the payload molecule is pIRES2DsRED2-hOPG.

5. The composition of claim 2, wherein the expression vector includes the polynucleotide of SEQ ID NO: 1.

6. The composition of claim 2, wherein the expression vector encodes a polypeptide selected from the group consisting of the polypeptide of SEQ ID NO: 2, a polypeptide consisting essentially of residues 28 to 124 of SEQ ID NO: 2, a polypeptide consisting essentially of residues 124 to 185 of SEQ ID NO: 2, and a polypeptide consisting essentially of residues 28 to 185 of SEQ ID NO: 2.

7. The composition of any one of claims 1 to 6 for use in the treatment of a condition **characterized by** low bone density.

8. The composition of any one of claims 1 to 6 for use in the treatment of osteoporosis, periprosthetic osteolysis, disuse osteopenia, arterial calcification, osteolysis associated with tumor metastasis, bone cancer pain, juvenile Paget's disease, Gaucher disease, HIV, arthritis, thalasemia or inflammatory bowel disease.

9. The composition of any one of claims 1 to 6 for use in therapy by increasing oesteoprotegerin expression in a cell.

10. The composition of claim 9, wherein the composition comprises a delivery system, wherein the payload molecule is an expression vector comprising a control element operatively linked to an open reading frame encoding an osteoprotegerin or a functional equivalent thereof.

11. The composition of claim 9 or 10, wherein the cell is a macrophage, an osteoclast, an osteoclast precursor, an M cell of a Peyer's patch, a monocyte, a neutrophil, a dendritic cell, a Langerhans cell, a Kupffer cell, an alveolar phagocyte, a peritoneal macrophage, a milk macrophage, a microglial cell, an eosinophil, a granulocytes, a mesengial phagocyte or a synovial A cell.

12. An oral, buccal, sublingual, pulmonary or transmucosal dosage form comprising the composition of any one of claims 1 to 6 and a pharmaceutically acceptable excipient for use in treating an osteoprotegerin-responsive condition.

13. The dosage form of claim 12, wherein the condition is osteoporosis, periprostheticosteolysis, disuse osteopenia, arterial calcification, osteolysis associated with tumor metastasis, bone cancer pain, juvenile Paget's disease, Gaucher disease, HIV, arthritis, thalasemia or inflammatory bowel disease.

14. A payload molecule containing composition of any one of claims 1 to 6 for use in:
a) a method of increasing osteoprotegerin expression in a cell; or
b) the treatment of a condition **characterized by** low bone density.

15. A payload molecule containing composition of any one of claims 1 to 6 for the use of claim 14, wherein the method of increasing osteoprotegerin expression in a cell includes the step of expressing an osteoprotegerin in the cell.

16. A payload molecule containing composition of any one of claims 1 to 6 for the use according to claim 14, wherein the medicament comprises the composition of claim 10 or claim 11.

17. A method of making an osteoprotegerin delivery system comprising the step of:
contacting a payload molecule that comprises a nucleic acid selected from the group consisting of an oligonucleotide, mRNA, a recombinant DNA construct, a linear DNA fragment, a blocked linear DNA fragment, and a mixture thereof, that encodes an anti-osteoclastogenic protein,
with a payload trapping molecule selected from the group consisting of chitosan, polyethylenimine, poly-L-lysine, hexadecyltrimethylammoniumbromide and mixtures thereof;
and a carrier selected from a yeast glucan particle or a yeast glucan-mannan particle, wherein the carrier is an extracted yeast cell wall defining an internal space, the cell wall comprising 6 to 90 weight percent beta-glucan, wherein the payload trapping molecule is contained within the internal space defined by the carrier, and wherein the payload trapping molecule is present for facilitating retention of the payload molecule within the carrier.

## Patentansprüche

1. Eine Zusammensetzung, umfassend:
ein Payloadmolekül, das eine Nukleinsäure umfasst, die aus der Gruppe ausgewählt ist, bestehend aus einem Oligonukleotid, mRNA, einem rekombinanten DNA-Konstrukt, einem linearen DNA-Fragment, einem blockierten linearen DNA-Fragment und einem Gemisch davon, und ein anti-osteoklastogenes Protein codiert; ein Payload-Fangmolekül, das aus der Gruppe ausgewählt ist, bestehend aus Chitosan, Polyethylenimin, Poly-L-Lysin, Hexadecyltrimethylammoniumbromid und Gemischen davon; und einen Träger, der aus einem Hefeglucan-Partikel oder einem Hefeglucanmannan-Partikel ausgewählt ist, wobei der Träger eine extrahierte Hefezellwand ist, die einen Innenraum umgrenzt, wobei die Zellwand 6 bis 90 Gew.-% beta-Glucan umfasst, wobei das Payload-Fangmolekül innerhalb des von dem Träger umgrenzten Innenraums eingeschlossen ist und wobei das Payload-Fangmolekül zur Förderung der Retention des Payloadmoleküls innerhalb des Trägers vorhanden ist.

2. Die Zusammensetzung nach Anspruch 1, wobei das anti-osteoklastogene Protein OPG oder ein funktionelles Äquivalent davon ist.

3. Die Zusammensetzung nach Anspruch 1, wobei das rekombinante DNA-Konstrukt ein Expressionsvektor ist, umfassend ein Kontrollelement, das an einen offenen Leserahmen funktionsfähig gebunden ist, der ein Osteoprotegerin oder ein funktionelles Äquivalent davon codiert.

4. Die Zusammensetzung nach Anspruch 1, wobei das Payloadmolekül pIRES2DsRED2-hOPG ist.

5. Die Zusammensetzung nach Anspruch 2, wobei der Expressionsvektor das Polynukleotid von SEQ ID NR: 1 umfasst.

6. Die Zusammensetzung nach Anspruch 2, wobei der Expressionsvektor ein Polypeptid codiert, das aus der Gruppe ausgewählt ist, bestehend aus dem Polypeptid von SEQ ID NR: 2, einem Polypeptid, bestehend im Wesentlichen aus Resten 28 bis 124 von SEQ ID NR: 2, einem Polypeptid, bestehend im Wesentlichen aus Resten 124 bis 185 von SEQ ID NR: 2, und einem Polypeptid, bestehend im Wesentlichen aus Resten 28 bis 185 von SEQ ID NR: 2.

7. Die Zusammensetzung nach einem der Ansprüche 1 bis 6 zur Verwendung in der Behandlung eines Zustandes, der durch geringe Knochendichte **gekennzeichnet** ist.

8. Die Zusammensetzung nach einem der Ansprüche 1 bis 6 zur Verwendung in der Behandlung von Osteoporose, periprosthetischer Osteolyse, Inaktivitätsosteopenie, arterieller Calcifizierung, mit Tumormetastase assoziierter Osteolyse, Knochenkrebsschmerz, juveniler Paget-Krankheit, Gaucher-Krankheit, HIV, Arthritis, Thalassämie oder entzündlicher Darmkrankheit.

9. Die Zusammensetzung nach einem der Ansprüche 1 bis 6 zur therapeutischen Verwendung durch Steigerung der Osteoprotegerinexpression in einer Zelle.

10. Die Zusammensetzung nach Anspruch 9, wobei die Zusammensetzung ein Abgabesystem umfasst, wobei das Payloadmolekül ein Expressionsvektor ist, umfassend ein Kontrollelement, das an einen offenen Leserahmen funktionsfähig gebunden ist, der ein Osteoprotegerin oder ein funktionelles Äquivalent davon codiert.

11. Die Zusammensetzung nach Anspruch 9 oder 10, wobei die Zelle ein Makrophage, ein Osteoklast, ein Osteoklasten-Vorläufer, eine M-Zelle eines Peyer-Plaques, ein Monocyt, ein neutrophiler Granulocyt, eine dendritische Zelle, eine Langerhans-Zelle, eine Kupffer-Zelle, ein Alveolarphagocyt, ein Peritonealmakrophage, ein Milchmakrophage, eine Mikrogliazelle, ein eosinophiler Granulocyt, ein Granulocyt, ein mesengialer Phagocyt oder eine synoviale A-Zelle ist.

12. Eine orale, bukkale, sublinguale, pulmonäre oder transmukosale Dosierungsform, umfassend die Zusammensetzung nach einem der Ansprüche 1 bis 6 und einen pharmazeutisch verträglichen Arzneimittelträger zur Verwendung in der Behandlung eines auf Osteoprotegerin ansprechenden Zustandes.

13. Die Dosierungsform nach Anspruch 12, wobei der Zustand Osteoporose, periprosthetische Osteolyse, Inaktivitätsosteopenie, arterielle Calcifizierung, mit Tumormetastase assoziierte Osteolyse, Knochenkrebsschmerz, juvenile Paget-Krankheit, Gaucher-Krankheit, HIV, Arthritis, Thalassämie oder entzündliche Darmkrankheit ist.

14. Eine Payloadmolekül-haltige Zusammensetzung nach einem der Ansprüche 1 bis 6 zur Verwendung in:
a) einem Verfahren zur Steigerung der Osteoprotegerinexpression in einer Zelle; oder
b) der Behandlung eines Zustandes, der durch geringe Knochendichte **gekennzeichnet** ist.

15. Eine Payloadmolekül-haltige Zusammensetzung nach einem der Ansprüche 1 bis 6 für die Verwendung nach Anspruch 14, wobei das Verfahren zur Steigerung der Osteoprotegerinexpression in einer Zelle den Schritt der Expression eines Osteoprotegerins in der Zelle umfasst.

16. Eine Payloadmolekül-haltige Zusammensetzung nach einem der Ansprüche 1 bis 6 für die Verwendung nach Anspruch 14, wobei das Arzneimittel die Zusammensetzung nach Anspruch 10 oder Anspruch 11 umfasst.

17. Ein Verfahren zur Bildung eines Osteoprotegerin-Abgabesystems, umfassend den Schritt:
Inkontaktbringen eines Payloadmoleküls, das eine Nukleinsäure umfasst, die aus der Gruppe ausgewählt ist, bestehend aus einem Oligonukleotid, mRNA, einem rekombinanten DNA-Konstrukt, einem linearen DNA-Fragment, einem blockierten linearen DNA-Fragment und einem Gemisch davon, und ein anti-osteoklastogenes Protein codiert;
mit einem Payload-Fangmolekül, das aus der Gruppe ausgewählt ist, bestehend aus Chitosan, Polyethylenimin, Poly-L-Lysin, Hexadecyltrimethylammoniumbromid und Gemischen davon; und einem Träger, der aus einem Hefeglucan-Partikel oder einem Hefeglucanmannan-Partikel ausgewählt ist, wobei der Träger eine extrahierte Hefezellwand ist, die einen Innenraum umgrenzt, wobei die Zellwand 6 bis 90 Gew.-% beta-Glucan umfasst, wobei das Payload-Fangmolekül innerhalb des von dem Träger umgrenzten Innenraums eingeschlossen ist und wobei das Payload-Fangmolekül zur Förderung der Retention des Payloadmoleküls innerhalb des Trägers vorhanden ist.

## Revendications

1. Composition comprenant :
une molécule à transporter qui comprend un acide nucléique choisi dans le groupe constitué par un oligonucléotide, un ARNm, un produit d'assemblage d'ADN recombinant, un fragment d'ADN linéaire, un fragment d'ADN linéaire bloqué ainsi qu'un mélange de ceux-ci, qui code pour une protéine anti-ostéoclastogène ;
une molécule de piégeage de la molécule à transporter, choisie dans le groupe constitué par le chitosane, la polyéthylène imine, la poly-L-lysine, le bromure d'hexadécyltriméthylammonium ainsi que des mélanges de ceux-ci ; et
un véhicule choisi parmi une particule de glucane de levure ou une particule de glucane-mannane de levure, lequel véhicule est une paroi cellulaire de levure extraite qui définit un espace intérieur, la paroi cellulaire comprenant de 6 à 90 pour cent en poids de bêta-glucane, dans laquelle la molécule de piégeage de la molécule à transporter est contenue à l'intérieur de l'espace intérieur défini par le véhicule et dans laquelle la molécule de piégeage de la molécule à transporter est présente afin de faciliter la rétention de la molécule à transporter à l'intérieur du véhicule.

2. Composition selon la revendication 1, dans laquelle la protéine anti-ostéoclastogène est l'OPG ou un équivalent fonctionnel de celle-ci.

3. Composition selon la revendication 1, dans laquelle le produit d'assemblage d'ADN recombinant est un vecteur d'expression qui comprend un élément de contrôle lié de manière opérante à un cadre de lecture ouvert qui code pour une ostéoprotégérine ou pour un équivalent fonctionnel de celle-ci.

4. Composition selon la revendication 1, dans laquelle la molécule à transporter est la pIRES2DsRED2-hOPG.

5. Composition selon la revendication 2, dans laquelle le vecteur d'expression comprend le polynucléotide de SEQ ID NO : 1.

6. Composition selon la revendication 2, dans laquelle le vecteur d'expression code pour un polypeptide choisi dans le groupe constitué par le polypeptide de SEQ ID NO : 2, un polypeptide essentiellement constitué des résidus 28 à 124 de la SEQ ID NO : 2, un polypeptide essentiellement constitué des résidus 124 à 185 de la SEQ ID NO : 2, et un polypeptide essentiellement constitué des résidus 28 à 185 de la SEQ ID NO : 2.

7. Composition selon l'une quelconque des revendications 1 à 6, destinée à être utilisée dans le traitement d'une condition **caractérisée par** une faible densité osseuse.

8. Composition selon l'une quelconque des revendications 1 à 6, destinée à être utilisée dans le traitement de l'ostéoporose, de l'ostéolyse périprothétique, de l'ostéopénie de non-utilisation, de la calcification artérielle, de l'ostéolyse associée à une métastase tumorale, de la douleur due au cancer des os, de la maladie de Paget juvénile, de la maladie de Gaucher, du VIH, de l'arthrite, de la thalassémie ou de la maladie entérique inflammatoire.

9. Composition selon l'une quelconque des revendications 1 à 6, destinée à être utilisée dans une thérapie par augmentation de l'expression d'ostéoprotégérine dans une cellule.

10. Composition selon la revendication 9, dans laquelle la composition comprend un système de délivrance, dans laquelle la molécule à transporter est un vecteur d'expression qui comprend un élément de contrôle lié de manière opérante à un cadre de lecture ouvert codant pour une ostéoprotégérine ou un équivalent fonctionnel de celle-ci.

11. Composition selon la revendication 9 ou la revendication 10, dans laquelle la cellule est un macrophage, un ostéoclaste, un précurseur d'ostéoclaste, une cellule M d'une plaque de Peyer, un monocyte, un neutrophile, une cellule dendritique, une cellule de Langerhans, une cellule de Kupffer, un phagocyte alvéolaire, un macrophage péritonéal, un macrophage du lait, une cellule microgliale, un éosinophile, un granulocyte, un phagocyte mésangial ou une cellule A synoviale.

12. Forme galénique pour l'administration par voie orale, buccale, sublinguale, pulmonaire ou transmucosale, comprenant une composition selon l'une quelconque des revendications 1 à 6 ainsi qu'un excipient pharmaceutiquement acceptable et destinée à être utilisée dans le traitement d'une condition qui réagit à l'ostéoprotégérine.

13. Forme galénique selon la revendication 12, dans laquelle la condition est l'ostéoporose, l'ostéolyse périprothétique, l'ostéopénie de non-utilisation, la calcification artérielle, l'ostéolyse associée à une métastase tumorale, la douleur due au cancer des os, la maladie de Paget juvénile, la maladie de Gaucher, le VIH, l'arthrite, la thalassémie ou la maladie entérique inflammatoire.

14. Composition contenant une molécule à transporter selon l'une quelconque des revendications 1 à 6, destinée à être utilisée :
a) dans un procédé d'augmentation de l'expression d'ostéoprotégérine dans une cellule ; ou
b) dans le traitement d'une condition **caractérisée par** une faible densité osseuse.

15. Composition contenant une molécule à transporter selon l'une quelconque des revendications 1 à 6 et destinée à être utilisée selon la revendication 14, dans laquelle le procédé d'augmentation de l'expression d'ostéoprotégérine dans une cellule comprend l'étape consistant à exprimer une ostéoprotégérine dans la cellule.

16. Composition contenant une molécule à transporter selon l'une quelconque des revendications 1 à 6 et destinée à être utilisée selon la revendication 14, dans laquelle le médicament comprend une composition selon la revendication 10 ou la revendication 11.

17. Procédé de production d'un système de délivrance d'ostéoprotégérine, comprenant l'étape consistant à :
mettre une molécule à transporter, comprenant un acide nucléique choisi dans le groupe constitué par un oligonucléotide, un ARNm, un produit d'assemblage d'ADN recombinant, un fragment d'ADN linéaire, un fragment d'ADN linéaire bloqué, ainsi qu'un mélange de ceux-ci, qui code pour une protéine anti-ostéoclastogène, en contact
avec une molécule de piégeage de la molécule à transporter, choisie dans le groupe constitué par le chitosane, la polyéthylène imine, la poly-L-lysine, le bromure d'hexadécyltriméthylammonium ainsi que des mélanges de ceux-ci ;
et avec un véhicule choisi parmi une particule de glucane de levure ou une particule de glucane-mannane de levure, lequel véhicule est une paroi cellulaire de levure extraite qui définit un espace intérieur, la paroi cellulaire comprenant de 6 à 90 pour cent en poids de bêta-glucane, dans laquelle la molécule de piégeage de la molécule à transporter est contenue à l'intérieur de l'espace intérieur défini par le véhicule et dans laquelle la molécule de piégeage de la molécule à transporter est présente afin de faciliter la rétention de la molécule à transporter à l'intérieur du véhicule.
